(19) Europäisches Patentamt
European Patent Office
Office européen des brevets

(11) EP 4 327 831 A1

(12) EUROPEAN PATENT APPLICATION
published in accordance with Art. 153(4) EPC

(43) Date of publication:
**28.02.2024 Bulletin 2024/09**

(21) Application number: **22791751.5**

(22) Date of filing: **20.04.2022**

(51) International Patent Classification (IPC):
***A61K 47/68*** *(2017.01)* ***A61K 39/395*** *(2006.01)*
***A61K 45/00*** *(2006.01)* ***A61K 47/60*** *(2017.01)*
***A61K 47/69*** *(2017.01)* ***A61K 51/00*** *(2006.01)*
***A61K 51/02*** *(2006.01)* ***A61K 51/10*** *(2006.01)*
***A61P 35/00*** *(2006.01)* ***C07K 4/00*** *(2006.01)*
***C07K 16/28*** *(2006.01)*

(52) Cooperative Patent Classification (CPC):
**A61K 39/395; A61K 45/00; A61K 47/60; A61K 47/68; A61K 47/69; A61K 51/00; A61K 51/02; A61K 51/10; A61P 35/00; C07K 4/00; C07K 16/28**

(86) International application number:
**PCT/JP2022/018246**

(87) International publication number:
**WO 2022/224980 (27.10.2022 Gazette 2022/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.04.2021 JP 2021071320**
**28.02.2022 JP 2022030389**

(71) Applicant: **Nihon Medi-Physics Co., Ltd.**
**Tokyo 136-0075 (JP)**

(72) Inventors:
- **KAWATANI, Minoru**
**Tokyo 136-0075 (JP)**
- **TAKENAKA, Fumiaki**
**Tokyo 136-0075 (JP)**
- **HANADA, Takahisa**
**Tokyo 136-0075 (JP)**
- **TONOYA, Gota**
**Tokyo 136-0075 (JP)**
- **TAKEDA, Takuya**
**Tokyo 136-0075 (JP)**

(74) Representative: **J A Kemp LLP**
**80 Turnmill Street**
**London EC1M 5QU (GB)**

Remarks:
The complete document including Reference Table(s) and the Sequence Listing(s) can be downloaded from the EPO website

# (54) RADIOACTIVE COMPLEX OF ANTI-CD20 ANTIBODY, AND RADIOPHARMACEUTICAL

(57) The present invention aims to provide a conjugate having stability improved more than conventional conjugates without impairing the efficacy. The conjugate of the present invention is a conjugate of an anti-CD20 antibody and a chelating agent, wherein the chelating agent is chelated with a metal radionuclide, the anti-CD20 antibody and the chelating agent are linked by a linker (L), and the linker (L) does not contain a thiourea bond.

[Fig. 4]
efficacy evaluation ($^{225}$Ac therapy experiment)

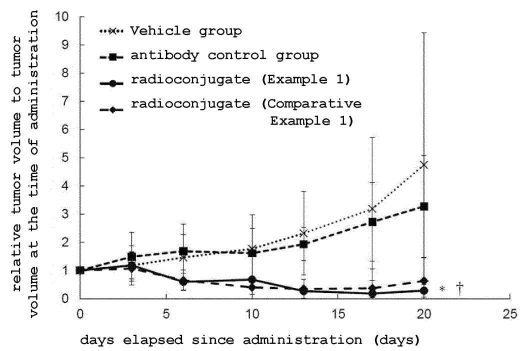


EP 4 327 831 A1

## Description

[Technical Field]

The present invention relates to a radioconjugate of an anti-CD20 antibody, and a radiopharmaceutical.

[Background Art]

CD20 is a transmembrane phosphorylated protein with a molecular weight of 33 to 37 kDa that is expressed on the surface of human B lymphocytes, and is expressed in many malignant B cells in addition to normal B cells in peripheral blood, spleen, tonsils, and bone marrow.

As an anti-CD20 antibody, rituximab is known. Rituximab is a monoclonal antibody (Patent Literature 3) consisting of a human-mouse chimeric antibody derived from mouse antibody 2B8 obtained by immunizing an SB cell line, which is a human B cell. Rituximab preparations are used as anticancer agents and immunosuppressants as one of the molecular targeted therapeutic drugs.

It is known that rituximab is an antibody used in ADCs (Antibody Drug Conjugates). Examples of ADC using rituximab include a medicament in which payload (drug), which is a chemotherapeutic agent, is covalently bonded to an antibody via a linker.

Antibody drugs have high target selectivity and relatively few side effects, but the efficacy thereof is sometimes insufficient. Chemotherapeutic agents as one type of payload have strong efficacy, but their low target selectivity increases the minimum effective dose necessary for killing cancer cells, and decreases the maximum tolerated dose because the dose cannot be increased much from the aspect of toxicity, thus causing a problem of narrow range of the therapeutic dosage.

According to ADC, higher amounts of chemotherapeutic agents can be selectively delivered to cancer cells. This is expected to result in wider therapeutic dosage ranges because lower doses achieve effects, chemotherapeutic agents that reach normal cells decrease, and the maximum tolerated doses increase.

One approach to ADC is a radioimmunoconjugate. In radioimmunoconjugates, radionuclides are used instead of payloads.

Patent Literature 1 relates to an ADC of anti-CD20 antibody, and discloses that the anti-CD20 antibody is labeled with RI. Patent Literature 2 relates to an ADC of anti-CD20 antibody (rituximab) and describes a drug in which a payload is covalently bonded to the antibody via a linker, and describes DOTA-Bn-thiourea linker as the linker.

[Citation List]

[Patent Literature]

[PTL 1]
WO 2004/032828
[PTL 2]
JP-A-2006-511532
[PTL 3]
US Patent No. 5736137

[Summary of Invention]

However, it has been clarified from the findings of the present inventors that conventional radioconjugates of the anti-CD20 antibody have problems such as low stability.

Patent Literature 1 and Patent Literature 2 do not disclose or suggest the problem of anti-CD20 antibody that forms a thiourea bond.

One embodiment of the present invention is a conjugate of an anti-CD20 antibody and a chelating agent, wherein the chelating agent is chelated with a metal radionuclide, the anti-CD20 antibody and the chelating agent are linked by a linker (L), and the linker (L) does not contain a thiourea bond.

Another embodiment of the present invention is a radiopharmaceutical containing the above-mentioned conjugate as an active ingredient.

In addition, another embodiment of the present invention is a radiopharmaceutical containing a conjugate of a chelating agent chelated with a metal radionuclide and an anti-CD20 antibody as active ingredients, wherein the linkage

between the anti-CD20 antibody and the chelating agent does not contain a thiourea bond, and the conjugate has a radiochemical purity of not less than 90% when stored at room temperature for 7 days.

The "linkage" in the present invention means both direct connection and indirect connection, unless otherwise specified.

According to the present invention, a radioconjugate of an anti-CD20 antibody is provided which has stability improved more than conventional conjugates without impairing the efficacy.

[Brief Description of Drawings]

[Fig. 1]
Fig. 1 shows the amino acid sequence of the rituximab light chain.
[Fig. 2-1]
Fig. 2-1 shows the amino acid sequence of the rituximab heavy chain.
[Fig. 2-2]
Fig. 2-2 shows the amino acid sequence of the rituximab heavy chain.
[Fig. 2-3]
Fig. 2-3 shows the amino acid sequence of the rituximab heavy chain.
[Fig. 3]
A graph showing evaluation results of the antigen-binding activities of radioconjugates prepared as described in Example 1 and Comparative Example 1. The vertical axis indicates values obtained by normalizing the value obtained by dividing the count value in ROI set on the tumor section used by the area of the ROI, with the value obtained by dividing the count value of the standard radiation source by the area of the ROI. The horizontal axis indicates the cell type of the tumor section used for evaluation. The graph represents the mean±standard deviation of ROI set in each sample (n=10).
[Fig. 4]
A graph showing changes in tumor volume over time in tumor-bearing mice of each radioconjugate (Example 1) group, radioconjugate (Comparative Example 1) group, an antibody control group, and a Vehicle group (n=6). The vertical axis indicates relative values when the tumor volume at the time of administration of each medicament is set to 1, and the horizontal axis indicates the number of days elapsed since administration of each medicament. The graph represents the mean±standard deviation of tumor volume in each group, "*" is the time point when a significant difference ($p<0.05$) was observed from the antibody control group, and "†" is the time point when a significant difference ($p<0.05$) was observed from the Vehicle group.
[Fig. 5]
A graph showing evaluation results of the antigen binding activity of radioconjugates prepared as described in Example 3 and Comparative Example 2. The vertical axis indicates values obtained by normalizing the value obtained by dividing the count value in ROI set on the tumor section used by the area of the ROI, with the value obtained by dividing the count value of the standard radiation source by the area of the ROI. The horizontal axis indicates the cell type of the tumor section used for evaluation. The graph shows the mean±standard deviation of ROI set in each sample (n=10).
[Fig. 6]
A graph showing the ratio of radioactivity accumulation in tumor to blood (n=3) in tumor-bearing mice administered with a radioconjugate prepared as described in Example 3. The vertical axis indicates the ratio of radioactivity accumulation in tumor to radioactivity accumulation in blood, and the horizontal axis indicates the cancerous tumor used for evaluation. The graph shows the mean±standard deviation of the ratio of radioactivity accumulation in tumor to blood.
[Fig. 7]
A graph showing the cytotoxic effect of $^{225}$Ac complex-labeled rituximab on SU-DHL-2 cells. Unlabeled rituximab and POLIVY (registered trademark) were added as comparison targets. The graphs show the mean±standard deviation in each group (n=4).

[Description of Embodiments]

(1) Radioconjugate

The present invention is directed to a conjugate of an anti-CD20 antibody and a chelating agent, wherein the chelating agent is chelated with a metal radionuclide, the anti-CD20 antibody and the chelating agent are linked by a

linker (L), and the linker (L) does not contain a thiourea bond (hereinafter to be also referred to as the radioconjugate of the present invention).

(1-1) Metal radionuclide

The metal radionuclide contained in the radioconjugate of the present invention is a radionuclide that emits α-ray, a radionuclide that emits β-ray, a radionuclide that emits positron, or a radionuclide that emits γ-ray. When the radioconjugate of the present invention is used for cancer therapy, it is preferable to use a radionuclide that emits α-ray or a radionuclide that emits β-ray. When the radioconjugate of the present invention is used for cancer diagnosis or detection, it is preferable to use a radionuclide that emits positron, or a radionuclide that emits γ-ray. Examples of the radionuclide that emits α-ray include Bi-212, Bi-213, Ac-225, and Th-227. Examples of the radionuclide that emits β-ray include Cu-64, Y-90, and Lu-177. Examples of the radionuclide that emits positron include Cu-64, Ga-68, Y-86, and Zr-89. Examples of the radionuclide that emits γ-ray include Tc-99m and In-111. The metal radionuclide to be contained in the radioconjugate of the present invention is more preferably Ga-68, Zr-89, Y-90, In-111, Lu-177, or Ac-225, further preferably Zr-89, Y-90, Lu-177, or Ac-225.

(1-2) Antibody

The antibody to be contained in the radioconjugate of the present invention is an immunoglobulin that specifically binds to CD20 (hereinafter also to be referred to as the antibody used in the present invention). The antibody to be used in the present invention may be a polyclonal antibody or a monoclonal antibody, preferably a monoclonal antibody. The origin of the antibody is not particularly limited, and examples include antibodies of non-human animals, non-human mammals, and humans, preferably antibodies of human, rat, mouse, and rabbit. When the antibody is derived from species other than human, it is preferably chimerized or humanized using well-known techniques. The antibody to be used in the present invention may be a chimeric antibody, a humanized antibody, or a human antibody. The antibody to be used in the present invention may be a bispecific antibody. The antibody to be used in the present invention is, for example, IgG, for example, IgG1, IgG2 (e.g., IgG2a or IgG2b), IgG3, or IgG4.

The antibody used in the radioconjugate of the present invention is more preferably rituximab or ibritumomab.

In the present specification, rituximab is specifically a humanized antibody containing the light chain amino acid sequence (SEQ ID NO: 1) shown in Fig. 1 and the heavy chain amino acid sequence (SEQ ID NO: 2) shown in Figs. 2-1 to 2-3.

Rituximab is used clinically as an anticancer drug indicated for CD20-positive B-cell non-Hodgkin's lymphoma and CD20-positive B-cell lymphoproliferative disease under immunosuppressive conditions and is available as Rituxan (registered trademark).

Ibritumomab is a mouse-type CD20 monoclonal antibody, and a drug labeled with yttrium (Y-90) is available as Zevalin (registered trademark).

(1-3) Chelating agent

In the present invention, the chelating agent is not particularly limited as long as it has a site in the structure thereof where metal radionuclide is coordinated. Examples of the chelating agent include CB-TE2A (1,4,8,11-Tetraazabicyclo[6.6.2]hexadecane-4,11-diacetic acid), CDTA (Cyclohexane-trans-1,2-diamine tetra-acetic acid), CDTPA (4-cyano-4-[[(dodecylthio)thioxomethyl]thio]-Pentanoic acid), DOTA (1,4,7,10-Tetraazacyclododecane-1,4,7,10-tetraacetic acid), DOTMA ((1R,4R,7R,10R)-α,α',α",α'"-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTPA (1,4,7,10-tetraazacyclododecane-1,4,7,10-tetra propionic acid), 1,4,7,10-tetrakis(pyridin-2-ylmethyl)-1,4,7,10-tetraazacyclododecane ($L^{py}$), DOTA-GA (α-(2-Carboxyethyl)-1,4,7,10-tetraazacyclododecane-1,4,7,10-tetraacetic acid), DOTP (((1,4,7,10-Tetraazacyclododecane-1,4,7,10-tetrayl)tetrakis(methylene))tetraphosphonic acid), DOTMP (1,4,7,10-Tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid), D02P (Tetraazacyclododecane dimethanephosphonic acid), Deferoxamine (DFO), DTPA (Glycine, N,N-bis[2-[bis(carboxymethyl)amino]ethyl]-), CHX-A"-DTPA (2,2'-((2-(((1S,2R)-2-(bis(carboxymethyl)amino)cyclohexyl) (carboxymethyl)amino)ethyl) azanediyl)diacetic acid), DTPA-BMA (5,8-Bis(carboxymethyl)-11-[2-(methylamino)-2-oxoethyl]-3-oxo-2,5,8,11-tetraazatridecan-13-oic acid), EDTA (2,2',2",2'"-(ethane-1,2-diylbis(azanetriyl))tetraacetic acid), NOTA (1,4,7-Triazacyclononane-1,4,7-triacetic acid), NOTP (1,4,7-Triazacyclononane-1,4,7-triyltris(methylenephosphonic acid)), TETPA (1,4,8,11-tetraazacyclotetradecane-1,4,8,11-tetrapropionic acid), TETA (1,4,8,11-Tetraazacyclotetradecane-N,N',N",N'"-tetraacetic acid), TTHA (3,6,9,12-Tetrakis(carboxymethyl)-3,6,9,12-tetraazatetradecanedioic acid), HEHA (1,2,7,10,13-hexaazacyclooctadecane-1,4,7,10,13,16-hexaacetic acid), 1,2-HOPO (N,N',N",N'"-tetra(1,2-dihydro-1-hydroxy-2-oxopyridine-6-carbonyl)-1,5,10,14-tetraazatetradecane), PEPA

(1,4,7,10,13-pentaazacyclopentadecane-N,N',N",N'",N""-pentaacetic acid), H4octapa (N,N'-bis(6-carboxy-2-pyridylmethyl)-ethylenediamine-N,N'-diacetic acid), H2bispa2 (6,6'-({9-hydroxy-1,5-bis(methoxycarbonyl)-2,4-di(pyridin-2-yl)-3,7-diazabicyclo[3.3.1]nonane-3,7-diyl}bis(-methylene))dipicolinic acid), H2dedpa (1,2-[{6-(carboxy)-pyridin-2-yl}-methylamino]ethane), H2macropa (6-(1,4,10,13-tetraoxa-7,16-diazacyclooctadecan-N,N'-methyl)picolinic acid), H5decapa (N,N"-bis(6-carboxy-2-pyridylmethyl)-diethylenetriamine-N,N',N"-triacetic acid), H6phospa (N,N'-(methylenephosphonate)-N,N'-[6-(methoxycarbonyl)pyridin-2-yl]-methyl-1,2-diaminoethane), HP-D03A (Hydroxypropyltetraazacyclododecanetriacetic acid), and porphyrin. A compound represented by the following formula (A) is preferred.

(A)

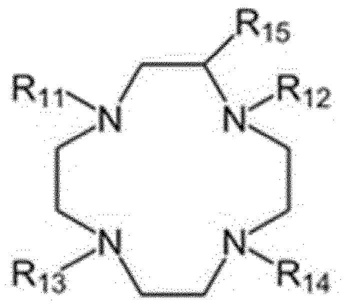

In the formula (A), $R_{11}$, $R_{12}$, $R_{13}$ and $R_{14}$ are each independently a group consisting of $-(CH_2)_pCOOH$, $-(CH_2)_pC_5H_5N$, $-(CH_2)_pPO_3H_2$, $-(CH_2)_pCONH_2$ or $-(CHCOOH)(CH_2)_pCOOH$, $R_{15}$ is a hydrogen atom, and p is an integer of not less than 0 and not more than 3.

The compound represented by the formula (A) is preferably a compound containing a structure derived from 1,4,7,10-tetraazacyclododecane-1,4,7,10-tetraacetic acid (DOTA), or a derivative thereof. Specifically, the compound can contain, in its structure, a structure derived from one chelating agent selected from DOTA (1,4,7,10-Tetraazacyclododecane-1,4,7,10-tetraacetic acid), DOTMA ((1R,4R,7R,10R)-α,α',α",α'"-tetramethyl-1,4,7,10-tetraazacyclododecane-1,4,7,10-tetraacetic acid), DOTAM (1,4,7,10-tetrakis(carbamoylmethyl)-1,4,7,10-tetraazacyclododecane), DOTPA (1,4,7,10-tetraazacyclododecane-1,4,7,10-tetrapropionic acid), 1,4,7,10-tetrakis(pyridin-2-ylmethyl)-1,4,7,10-tetraazacyclododecane ($L^{py}$), DOTA-GA (α-(2-Carboxyethyl)-1,4,7,10-tetraazacyclododecane-1,4,7,10-tetraacetic acid), DOTP (((1,4,7,10-Tetraazacyclododecane-1,4,7,10-tetrayl)tetrakis(methylene))tetraphosphonic acid), DOTMP (1,4,7,10-Tetraazacyclododecane-1,4,7,10-tetrakis(methylenephosphonic acid)), DOTA-4AMP (1,4,7,10-tetraazacyclododecane-1,4,7,10-tetrakis(acetamidomethylenephosphonic acid), and D02P (Tetraazacyclododecane dimethanephosphonic acid). More preferably, compounds represented by the following formulas (A-1) to (A-6) can be mentioned. The chelating agent to be used in the radioconjugate of the present invention is further preferably DOTA-GA (compound represented by the formula (A-6)). When the chelating agent used is DOTA-GA, the aforementioned chelating agent may be a stereoisomer (S-form, R-form) or a racemate. The stereoisomers of S-form and R-form may be mixed in any ratio.

(A-1)

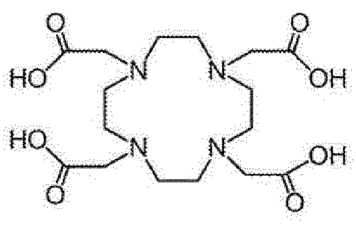

DOTA

(A-2)

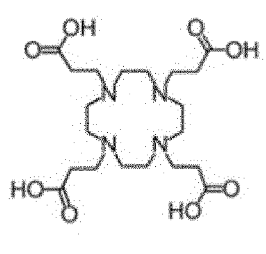

DOTPA

(A-3)

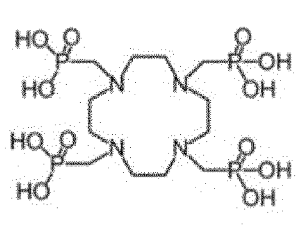

DOTMP

(A-4)

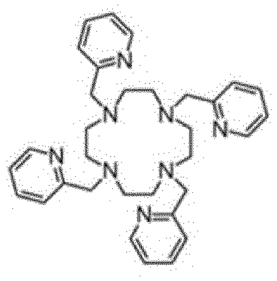

$L^{py}$

(A-5)

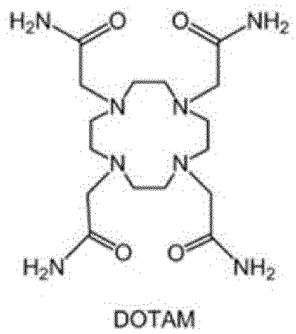

DOTAM

(A-6)

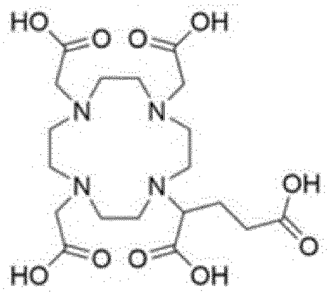

DOTA-GA

A chelating agent used in the present invention is linked with an anti-CD20 antibody via a linker (L). Preferably, the anti-CD20 antibody used in the present invention is site-specifically modified with peptide. In this case, a chelating agent used in the present invention is linked with a peptide via a linker (L). In the radioconjugate of the present invention, the chelating agent and the linker (L) are preferably connected by a covalent bond. Therefore, in the radioconjugate of the present invention, some groups in the compound of the aforementioned chelating agent may be substituted by groups that form covalent bonds with the linker (L). For example, when the chelating agent used in the present invention is a compound represented by the formula (A), $R_{12}$ or $R_{15}$ may be substituted by a group that forms a covalent bond with the linker (L). Preferably, when $R_{12}$ is substituted by a group that forms a covalent bond with the linker (L), $R_{15}$ is a hydrogen atom, when $R_{12}$ is a group consisting of $-(CH_2)_pCOOH$, $-(CH_2)_pC_5H_5N$, $-(CH_2)_pPO_3H_2$, $-(CH_2)_pCONH_2$, or $-(CHCOOH)(CH_2)_pCOOH$, $R_{15}$ is substituted by a group that forms a covalent bond with the linker (L).

The covalent bond between the chelating agent and the linker (L) only needs to be free of a thiourea bond, and is exemplified by a carbon-carbon bond, an amide bond, an ether bond, an ester bond, and the like.

The connection between the chelating agent and the linker (L) can be formed, for example, by the reaction of an N-hydroxysuccinimide ester (NHS) group of the following formula (A-7) or (A-8), or a 2,6-dioxotetrahydro-2H-pyranyl group of the following formula (A-9), with the primary amine of linker (L) .

(A-7)

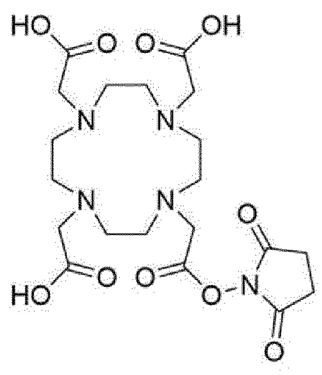

DO3A-NHS

(A-8)

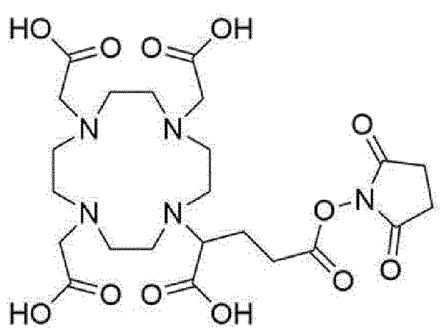

DOTA-GA-NHS

(A-9)

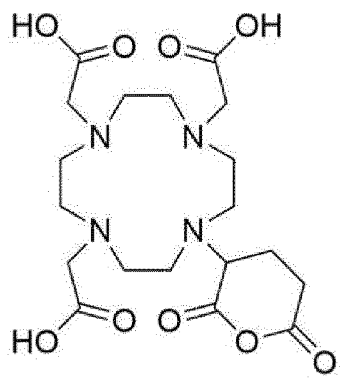

DOTA-GA-anhydride

(1-4) Antibody-modification peptide

In the present invention, the peptide is not particularly limited as long as it modifies the antibody site-specifically, preferably the Fc region site-specifically, more preferably the lysine residue in the Fc region of the antibody site-specifically. As a result, it is possible to maintain the activity of the antibody itself (antigen recognition action, neutralizing action, complement activating action and/or opsonin action).

The peptide to be used in the present invention may be a chain peptide or a cyclic peptide, and cyclic peptide is preferred. More preferably, it contains an amino acid sequence consisting of 13 to 17 amino acid residues represented by the following formula (i) (hereinafter, to be also referred to as "antibody-modification peptide"), and is modified with

a crosslinking agent. In the explanation of the formula (i), the left side of the paper surface of the amino acid sequence indicates the N-terminal side, and the right side of the paper surface of the amino acid sequence indicates the C-terminal side.

$$(Xa)\ \text{-}Xaa_1\text{-}\ (Xb)\ \text{-}Xaa_2\text{-}\ (Xc)\ \text{-}Xaa_3\text{-}\ (Xd) \qquad (i)$$

In the formula (i), Xa, Xb, Xc and Xd are each continuous

X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy a+b+c+d≤14,
$Xaa_1$ and $Xaa_3$ are each independently an amino acid residue derived from an amino acid having a thiol group in the side chain, or an amino acid residue derived from an amino acid having a haloacetyl group in the side chain, wherein either $Xaa_1$ or $Xaa_3$ is an amino acid residue derived from an amino acid having a thiol group, preferably $Xaa_1$ and $Xaa_3$ are linked to form a ring structure, and
$Xaa_2$ is a lysine residue, arginine residue, cysteine residue, aspartic acid residue, glutamic acid residue, 2-aminosuberic acid, or diamino propionic acid, preferably lysine residue, and $Xaa_2$ is modified with a crosslinking agent.

Examples of the amino acid residue that may be contained in X in the above-mentioned formula (i) include those derived from amino acids such as glycine, alanine, phenylalanine, proline, asparagine, aspartic acid, glutamic acid, arginine, histidine, serine, threonine, tyrosine, methionine and the like, and when a plurality of X is present, they may be an amino acid residue consisting of the same type of amino acid, or different types of amino acids.

In the formula (i), a, b, c and d are not particularly limited as long as they are numbers within the aforementioned range. From the aspect of the stability of binding between the peptide and anti-CD20 antibody, a+b+c+d≤14 is to be satisfied, and a is preferably an integer of not less than 1 and not more than 3, b is preferably an integer of not less than 1 and not more than 3, c is preferably an integer of not less than 3 and not more than 5, and d is preferably an integer of not less than 1 and not more than 3.

At least one of $Xaa_1$ and $Xaa_3$ is an amino acid residue derived from an amino acid having a thiol group in the side chain, and $Xaa_1$ and $Xaa_3$ may be the same or different. Examples of the amino acid having a thiol group in the side chain include cysteine and homocysteine. Such amino acid residues are preferably bonded by a disulfide bond, or a sulfide group is preferably bonded thereto via a structure shown by the following formula (4). In the formula (4), the wavy line indicates the binding part with the sulfide group.

(4) 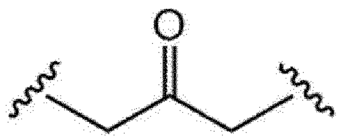

Instead of the aforementioned combination of $Xaa_1$ and $Xaa_3$, one of $Xaa_1$ and $Xaa_3$ may be an amino acid residue derived from an amino acid having a thiol group in the side chain, and the other may be an amino acid residue derived from an amino acid having a haloacetyl group in the side chain. These are bonded via a thioether bond. The terminal of the haloacetyl group is substituted with a halogen such as iodine or the like, and the halogen is eliminated by a reaction with the thiol group in the other side chain, whereby a thioether bond is formed.

Specific examples of the amino acid sequence of the antibody-modification peptide represented by the formula (i) include the peptides described in WO 2016/186206, WO 2017/217347 and WO 2018/230257, and these can also be used.

Preferably, the antibody-modification peptide to be used in the present invention is an amino acid sequence consisting of 13 to 17 amino acid residues represented by the following formula (i)'.

$$(X_{1\text{-}3})\ \text{-C-}\ (Xaa_3')\ \text{-}\ (Xaa_4')\ \text{-H-}\ (Xaa_1')\ \text{-G-}\ (Xaa_2')\ \text{-L-V-W-C-}\ (X_{1\text{-}3}) \qquad (i)'$$

In the formula (i)', each X is independently any amino acid residue other than cysteine,

C is a cysteine residue,
H is a histidine residue,
$Xaa_1'$ is a lysine residue, a cysteine residue, an aspartic acid residue, a glutamic acid residue, a 2-aminosuberic

acid, or a diamino propionic acid,
G is a glycine residue,
$Xaa_2$' is a glutamic acid residue or an asparagine residue,
L is a leucine residue,
V is a valine residue,
W is a tryptophan residue,
$Xaa_3$' is an alanine residue, a serine residue, or a threonine residue, and
$Xaa_4$' is a tyrosine residue or a tryptophan residue.

In the above-mentioned formula (i)', N-terminal or C-terminal $X_{1-3}$ means that any 1 to 3 amino acid residues X other than cysteine (C or Cys) are independently consecutive, and is a sequence consisting of the same or different amino acid residues, preferably all three different amino acid residues.

Among these, the amino acid sequence of the antibody-modification peptide preferably has any one of the following sequences (1) to (14), more preferably the following sequence (1), (2), (13) or (14). In the following amino acid sequences (1) to (14), ($Xaa_2$) is a lysine residue, a cysteine residue, an aspartic acid residue, a glutamic acid residue, a 2-aminosuberic acid, or a diamino propionic acid, preferably a lysine residue, ($Xaa_2$) is preferably modified with a crosslinking agent, and ($Xaa_1$) and ($Xaa_3$) are each a homocysteine residue. In the following amino acid sequences (1) to (14), the amino acids other than ($Xaa_1$), ($Xaa_2$) and ($Xaa_3$) are indicated by one-letter abbreviations.

(1) DCAYH($Xaa_2$)GELVWCT (SEQ ID NO: 3)
(2) GPDCAYH($Xaa_2$)GELVWCTFH (SEQ ID NO: 4)
(3) RCAYH($Xaa_2$)GELVWCS (SEQ ID NO: 5)
(4) GPRCAYH($Xaa_2$)GELVWCSFH (SEQ ID NO: 6)
(5) SPDCAYH($Xaa_2$)GELVWCTFH (SEQ ID NO: 7)
(6) GDDCAYH($Xaa_2$)GELVWCTFH (SEQ ID NO: 8)
(7) GPSCAYH($Xaa_2$)GELVWCTFH (SEQ ID NO: 9)
(8) GPDCAYH($Xaa_2$)GELVWCSFH (SEQ ID NO: 10)
(9) GPDCAYH($Xaa_2$)GELVWCTHH (SEQ ID NO: 11)
(10) GPDCAYH($Xaa_2$)GELVWCTFY (SEQ ID NO: 12)
(11) SPDCAYH($Xaa_2$)GELVWCTFY (SEQ ID NO: 13)
(12) SDDCAYH($Xaa_2$)GELVWCTFY (SEQ ID NO: 14)
(13) RGNCAYH($Xaa_2$)GQLVWCTYH (SEQ ID NO: 15)
(14) G($Xaa_1$)DCAYH($Xaa_2$)GELVWCT($Xaa_3$)H (SEQ ID NO: 16)

The peptide represented by the above-mentioned formula (i) or (i)', or the peptide having the sequences (1) to (14) preferably has a linker (L) introduced at the N-terminal and is amidated at the C-terminal. Furthermore, $Xaa_2$ (or a part corresponding to $Xaa_2$) of these peptides is modified with a crosslinking agent, which allows the peptides to covalently bind to the Fc region of the anti-CD20 antibody via the crosslinking agent. In formula (i)', the part corresponding to $Xaa_2$ is $Xaa_1$'.

A crosslinking agent can be appropriately selected by those of ordinary skill in the art, and can be a compound having at least two sites bindable to desired amino acids (e.g., lysine residue, cysteine residue, aspartic acid residue, glutamic acid residue, 2-aminosuberic acid, diaminopropionic acid, arginine residue, etc.). Examples thereof include, but are not limited to, a crosslinking agent preferably containing two or more succinimidyl groups such as DSG (disuccinimidyl glutarate), DSS (disuccinimidyl suberate), and the like, a crosslinking agent preferably containing two or more imide acid moieties such as DMA (dimethyl adipimidate·2HCl, dimethyl adipimidate dihydrochloride), DMP (dimethyl pimelimidate·2HCl, dimethyl pimelimidate dihydrochloride), DMS (dimethyl suberimidate·2HCl, dimethyl suberimidate dihydrochloride), and the like, and a crosslinking agent having an SS bond such as DTBP (dimethyl 3,3'-dithiobispropionimidate·2HCl, dimethyl 3,3'-dithiobispropionimidate dihydrochloride) and DSP (dithiobis(succinimidyl propionate)), and the like, and SBAP (succinimidyl 3-(bromoacetamido)propionate). A crosslinking agent containing a succinimidyl group such as DSS or DSG reacts with a primary amine present at the N-terminal of peptide. Therefore, by blocking the N-terminal and reacting with DSS or DSG, only the amino group of $Xaa_2$ can be specifically modified with DSS or DSG. For example, linker (L) may be previously introduced into the N-terminal of the antibody-modification peptide and then reacted with DSS or DSG. The anti-CD20 antibody is site-specifically modified with the peptide when the succinimidyl group of DSS or DSG reacts with Lys250 residue or Lys252 residue according to Eu numbering in the anti-CD20 antibody (for example, rituximab). These Lys residues are present in the Fc region of human IgG, and even if the antibody is anti-CD20 antibody other than rituximab, those skilled in the art can align the amino acid sequence of the antibody and identify the corresponding Lys residue.

(1-5) Linker (L)

Linker (L) is not particularly limited as long as it can link a chelating agent and a peptide in the radioconjugate of the present invention. Linker (L) to be used in the present invention is not particularly limited as long as it does not contain a thiourea bond. Examples thereof include substituted or unsubstituted alkyl group, substituted or unsubstituted heteroalkyl group, polyethylene glycol (PEG) group, peptides, sugar chain, disulfide group, amide group, combination of these and the like.

In the present specification, linker (L) is a general term for linkers used for the connection of an anti-CD20 antibody modified with a peptide and a chelating agent, and includes antibody-modification linker ($L_1$) and chelate linker ($L_2$). The antibody-modification linker ($L_1$), which is described in detail later, is introduced into the N-terminal side of the peptide described in (1-4), and the chelate linker ($L_2$), which is described in detail later, is introduced into the functional group of the chelating agent described in (1-3).

The linker (L) used in the present invention may contain a binding site formed by a click reaction, and preferably, the antibody-modification linker ($L_1$) and the chelate linker ($L_2$) are bound by a click reaction. In the present invention, it is preferred that a thiourea bond is not contained between the binding site formed by the click reaction and the chelating agent. In other words, it is preferred that the chelate linker ($L_2$) does not contain a thiourea bond. As used herein, the binding site formed by the click reaction is preferably a triazole skeleton-containing structure represented by the following formula (10a) or (10b) or a pyridazine skeleton-containing structure represented by the following formula (10c) can be considered. Since the formula (10a) and the formula (10b) are isomers, they may be contained at any ratio.

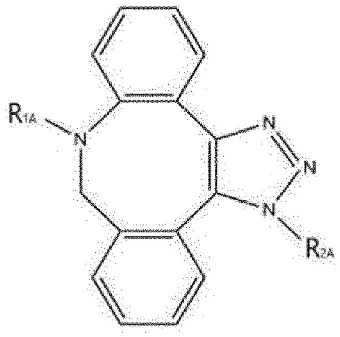
(1 0 a)

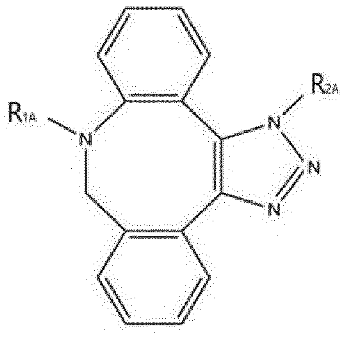
(1 0 b)

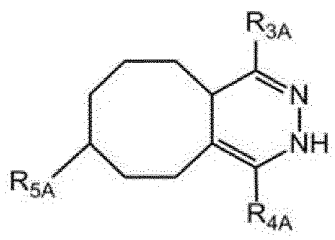
(1 0 c)

In the formula (10a) and the formula (10b), $R_{1A}$ is a linkage site with a chelating agent, and $R_{2A}$ is a linkage site with an antibody-modification peptide. In the formula (10c), one of $R_{3A}$ and $R_{4A}$ is a hydrogen atom, a methyl group, a phenyl group or a pyridyl group, and the other is a linkage site with a chelating agent, and $R_{5A}$ is a linkage site with an antibody-modification peptide. In the formula (10a), the formula (10b), and the formula (10c), the linkage site with the antibody-modification peptide is linked with the peptide via the antibody-modification linker ($L_1$), and the linkage site with the chelating agent is linked with the chelating agent via the chelate linker ($L_2$).

In the radioconjugate of the present invention, the antibody is site-specifically modified with a peptide, and the peptide and a chelating agent are linked via a linker (L). Thus, one molecule or two molecules of the chelating agent are conjugated to one molecule of the anti-CD20 antibody.

(1-6) Production method of conjugate

The radioconjugate of the present invention can be produced by two steps which are a conjugation step of conjugating a chelating agent and an anti-CD20 antibody, and a complex formation step of forming a complex of a metal radionuclide and a chelating agent. The conjugation step may be performed before the complex formation step or after the complex formation step.

In the conjugation step, the Fc region of an antibody is site-specifically modified with a chelating agent or linker (L) having the aforementioned antibody-modification peptide.

In the complex formation step, the chelating agent is chelated with a metal radionuclide (complex formation). The metal radionuclide used here is preferably used in a manner permitting ionization, more preferably in the form of an ion, from the viewpoint of increasing the complex formation efficiency. In the complex forming step, the order of addition of the metal radionuclide to the chelating agent does not matter as long as a complex can be formed with the metal radionuclide. For example, a solution in which radioactive metal ions are dissolved in a solvent mainly composed of water can be used as a radionuclide.

After complex formation, the obtained complex may be purified using a filtration filter, a membrane filter, a column filled with various fillers, chromatography or the like.

In the production method of the radioconjugate of the present invention, a conjugation step is preferably performed after the complex formation step.

In a more preferred embodiment, in complex formation step (A), a complex is formed between a metal radionuclide and a chelating agent having a first atomic group capable of click reaction as a substituent for enabling conjugate formation with the antibody. Then, in conjugation step (B), using an antibody-modification peptide shown by the aforementioned formula and an antibody-modification linker ($L_1$) having a second atomic group capable of click reaction, a click reaction is performed between the peptide-modification antibody in which Fc region is site-specifically modified and the chelating agent with a formed complex which is obtained in step (A) to obtain the radioconjugate of the present invention.

The steps (A) and (B) are described in detail below.

As the combination of the first atomic group and the second atomic group capable of click reaction, an appropriate combination is selected according to the type of the click reaction. For example, a combination of alkyne and azide, a combination of 1,2,4,5-tetrazine and alkene, and the like can be mentioned. In these atomic groups, the first atomic group has one of the above-mentioned atomic group combination, and the second atomic group has one atomic group which is different from the first atomic group of the above-mentioned atomic group combination. To achieve both the stability of the chelating agent and the antibody and the improvement of the binding efficiency thereof, the chelate linker ($L_2$) is preferably alkyne and the antibody-modification linker ($L_1$) is preferably azide, or the chelate linker ($L_2$) is preferably 1,2,4,5-tetrazine and the antibody-modification linker ($L_1$) is preferably alkene. Specific examples of the click reaction by such combinations of atomic groups include a Husgen cyclization addition reaction, an inverse electron-requested Diels-Alder reaction, and the like.

Specific examples of the combination of the atomic groups capable of click reaction include, as shown in the following formulas, a combination of an atomic group containing dibenzylcyclooctyne (DBCO) as alkyne of the first atomic group (the formula (1a)) and an atomic group containing an azide group as azide of the second atomic group (the formula (2a)), and a combination of an atomic group containing 1,2,4,5-tetrazine as the first atomic group (the formula (1b)) and an atomic group containing trans-cyclooctene (TCO) as alkene of the second atomic group (the formula (2b)). Preferred is the combination of the formula (1a) and the formula (2a).

(1a) 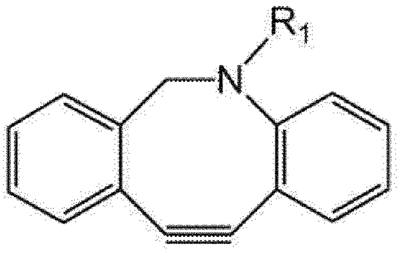

Dibenzylcyclooctyne

(2a) 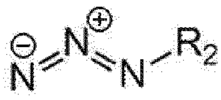

Azide

In the formula (1a), $R_1$ is a linkage site with a chelating agent, and in the formula (2a), $R_2$ is a linkage site with an antibody-modification peptide.

(1b) 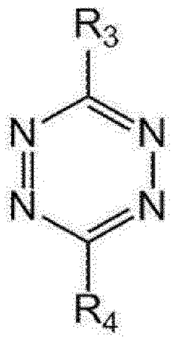

1,2,4,5-tetrazine

(2b) 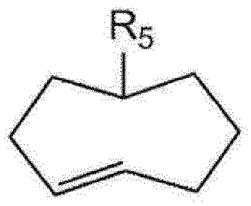

*trans*-cyclooctene

In the formula (1b), one of $R_3$ and $R_4$ is a linkage site with a chelating agent or an antibody-modification peptide, and the other is a hydrogen atom, a methyl group, a phenyl group or a pyridyl group. When the atomic group in the formula (1b) is linked to the chelating agent, $R_5$ is a linkage site to an antibody-modification peptide and when the atomic group in the formula (1b) is linked to the antibody-modification peptide, $R_5$ is a linkage site to the chelating agent.

When an atomic group containing dibenzylcyclooctyne (DBCO) represented by the above-mentioned formula (1a) as alkyne of the first atomic group is used, various commercially available DBCO reagents can be mentioned. Specifically, for example, DBCO-C6-Acid, Dibenzylcyclooctyne-Amine, Dibenzylcyclooctyne Maleimide, DBCO-PEG acid, DBCO-PEG-Alcohol, DBCO-PEG-amine, DBCO-PEG-NH-Boc, Carboxyrhodamine-PEG-DBCO, Sulforhodamine-PEG-DBCO, TAMRA-PEG-DBCO, DBCO-PEG-Biotin, DBCO-PEG-DBCO, DBCO-PEG-Maleimide, TCO-PEG-DBCO, DBCO-mPEG and the like can be selected, and Dibenzylcyclooctyne Maleimide is preferably used.

In complex formation step (A), more preferably, a compound having a structure represented by the following formula (ii) is used.

A-B-C (ii)

In the formula (ii), A is the aforementioned chelating agent, and the generic term of B and C is a chelate linker ($L_2$)

In the formula (ii), B is represented by the following formula (iib).

(iib) 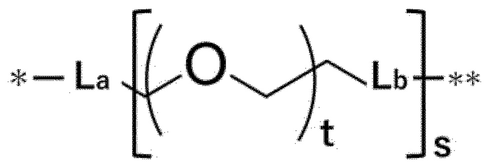

In the formula (iib), La and Lb are each independently a bond linker containing at least an amide bond and not less than 1 and not more than 50 carbon atoms, t is an integer of not less than 0 and not more than 30, s is 0 or 1, * is a binding site with A, and ** is a binding site with C.

In the formula (ii), C is either an alkyne derivative represented by the following formula (iic) or a tetrazine derivative represented by the formula (iid).

(iic) 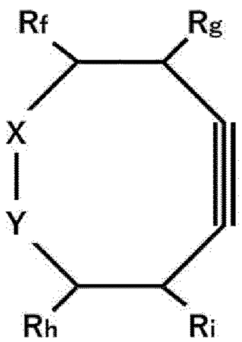

(iid) 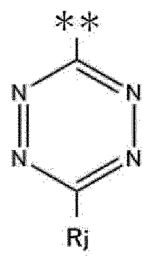

In the formula (iic), X is CHRk-** or N-**, Y is CHRk or C=O, Rk is independently a hydrogen atom or an alkyl group having not less than 1 and not more than 5 carbon atoms, when X is CHRk-** and Y is CHRk, then Rk moieties may be joined to form a cycloalkyl group, Rf, Rg, Rh and Ri are each independently a hydrogen atom, a halogen atom, or an alkyl group having not less than 1 and not more than 5 carbon atoms, Rf and Rg may be joined, or Rh and Ri may be joined to form a hydrocarbon ring, ** is a binding site with B, in the formula (iid), ** is a binding site with B, and Rj is a hydrogen atom, a methyl group, a phenyl group or a pyridyl group.

As the chelating agent used in step (A), a DOTA derivative of the above-mentioned formula (A) wherein $R_{11}$ to $R_{14}$ are $-(CH_2)_pCOOH$, p is 1, $R_{15}$ is a binding site with B; or DO3A derivative or DOTAGA derivative wherein $R_{11}$ to $R_{14}$ are - $(CH_2)_pCOOH$, p is 1, $R_{12}$ is a binding site (*) with B, and $R_{15}$ is a hydrogen atom is more preferred.

In the formula (ii), a DO3A-PEGt-DBCO wherein A is the above-mentioned DO3A, in B, La is a bond linker containing an amide bond and having not less than 1 and not more than 50 carbon atoms, s is 0 or 1, when s is 1, t is an integer of not less than 0 and not more than 30, Lb is a bond linker containing an amide bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is preferred.

In the formula (ii), a DOTAGA-PEGt-DBCO derivative wherein A is the above-mentioned DOTAGA derivative, in B, La is a bond linker containing an amide bond and having not less than 1 and not more than 50 carbon atoms, s is 0 or 1, when s is 1, t is an integer of not less than 0 and not more than 30, Lb is a bond linker containing an amide bond and having not less than 1 and not more than 50 carbon atoms, and C is an alkyne derivative represented by the formula (iic), wherein, in the formula (iic), X is N-**, Y is CHRk, Rk is a hydrogen atom, Rf and Rg are jointed to form a benzene ring, Rh and Ri are jointed to form a benzene ring, and ** is a binding site with B is preferred. More preferred is the following DOTAGA-DBCO.

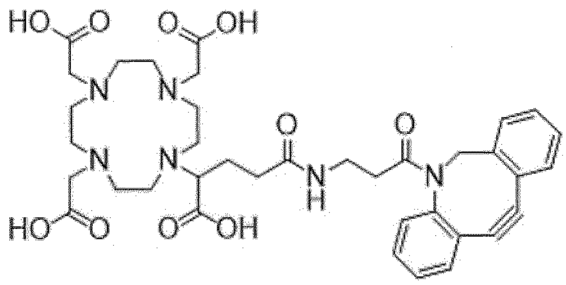

DOTAGA-DBCO

In the molar ratio of the chelating agent and metal radionuclide as chelate site/metal radionuclide, the lower limit is preferably not less than 10/1, more preferably not less than 100/1, further preferably not less than 500/1, and the upper limit is preferably not more than 10000/1, more preferably not more than 8000/1, further preferably not more than 7000/1. For example, the range of not less than 100/1 and not more than 7000/1 is preferred, and not less than 500/1 and not more than 7000/1 is more preferred.

The complex formation reaction is preferably performed in a solvent. As the solvent, water, saline, buffers such as sodium acetate buffer, ammonium acetate buffer, phosphate buffer, phosphate buffered saline, tris hydroxymethylaminomethane buffer (Tris buffer), 4-(2-hydroxyethyl)-1-piperazineethanesulfonic acid buffer (HEPES buffer), tetramethylammonium acetate buffer and the like, and the like can be used.

While the amount of the solvent is not particularly limited, from the aspect of practicality in the production step, the lower limit at the start of step (A) is not less than 0.01 mL, preferably not less than 0.1 mL, more preferably not less than 1.0 mL, further preferably not less than 10 mL, further more preferably not less than 100 mL, and upper limit is preferably not more than 1000 mL, more preferably not more than 100 mL, further preferably not more than 10 mL, further more preferably not more than 1.0 mL. For example, it is within the range of not less than 0.01 mL and not more than 100 mL.

As the concentration of the chelating agent in the reaction mixture of the complex formation reaction, from the aspect of the yield of the desired chelating agent, the lower limit at the start of step (A) is each independently preferably not less than 0.001 μmol/L, more preferably not less than 0.01 μmol/L, further preferably not less than 0.1 μmol/L, more preferably not less than 1 μmol/L, and the upper limit is preferably not more than 1000 μmol/L, more preferably not more than 100 μmol/L, further preferably not more than 10 μmol/L. For example, it is within the range of not less than 1 μmol/L and not more than 100 μmol/L.

The temperature of the complex formation reaction may be, for example, room temperature (25°C) or under heating conditions. To simultaneously achieve suppression of decomposition of the chelating agent and improvement of complex formation efficiency, the lower limit is preferably not less than 20°C, more preferably not less than 30°C, further preferably not less than 35°C, further more preferably not less than 37°C, particularly preferably not less than 45°C. The upper limit is preferably not more than 150°C, more preferably not more than 120°C, further preferably not more than 100°C, further more preferably not more than 90°C. For example, a range of not less than 30°C and not more than 100°C is preferred, and a range of not less than 35°C and not more than 90°C is more preferred.

The antibody to be used in step (B) is a peptide-modified antibody in which Fc region (constant region) of anti-CD20 antibody as described in detail in the above-mentioned "(1-2) Antibody" is site-specifically modified using the aforementioned antibody-modification peptide and an antibody-modification linker ($L_2$) having the second atomic group capable of click reaction.

The antibody-modification peptide can be produced using a combination of amino acids regardless of natural amino acids and unnatural amino acids, by subjecting to peptide synthesis methods such as liquid phase synthesis process, solid phase synthesis process, automatic peptide synthesis method, gene recombinant method, phage display method and the like. In the synthesis of the peptide, where necessary, the functional groups of the amino acids to be used may be protected. These methods can be performed according to the method described in, for example, WO 2017/217347 and WO 2018/230257.

The antibody-modification linker ($L_2$) may be one in which an antibody-modification peptide and a linker ($L_2$) represented by the following formula (S1) are bonded.

$$*-((L_i)_m-Z)_k-L_{ii}-AG2 \qquad (S1)$$

wherein * is a binding site with the N-terminal or C-terminal of peptide,

$L_i$ is a linker moiety of polyethylene glycol (PEG),
m is an integer of not less than 1 and not more than 50,
Z is a second linker moiety that binds $(L_i)_m$ and $L_{ii}$,
k is 0 or 1,
$L_{ii}$ is the second PEG linker moiety, and
AG2 is a second atomic group.

In the aforementioned formula (S1), the structure of Z is not particularly limited as long as it is a linker structure that binds $(L_i)_m$ and $L_{ii}$ to each other, and includes, for example, an amino acid sequence consisting of not less than 1 and not more than 5 amino acid residues. In this case, the amino acid sequence contained in Z preferably contains a cysteine residue, and is more preferably bonded to L2 via a thioether group formed by the bond between the thiol group of the cysteine residue and a maleimide group.

In the present invention, the polyethylene glycol (PEG) linker moiety constituting $L_{ii}$ preferably has the structure shown by the following formula (P2). In the formula (P2), n is an integer of preferably not less than 1 and not more than 50, more preferably not less than 1 and not more than 20, further preferably not less than 2 and not more than 10, further more preferably not less than 2 and not more than 6.

(P2) 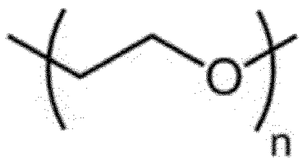

One end of the structure of the PEG linker moiety may be modified by a structure derived from a commercially available PEGylation reagent or a structure derived from a reagent generally used for PEGylation. Although not particularly limited, examples thereof include structures derived from diglycolic acid or a derivative thereof, and maleimide or a derivative thereof.

As a method for introducing the aforementioned second atomic group into an antibody-modification linker ($L_2$), an introduction method including obtaining an antibody-modification peptide having a desired amino acid sequence by the aforementioned method, dissolving the peptide in a solution containing a solubilizing agent and a reducing agent and, where necessary, an acid, adding an organic solvent solution of an atomic group containing an azide group or trans-cyclooctene (TCO) as the second atomic group to the solution, and stirring the mixture at room temperature can be mentioned.

When an atomic group containing an azide group is introduced as the second atomic group, the azide group is directly introduced into the N-terminal or C-terminal of a peptide by using a commercially available azide group-introducing reagent according to a conventional method, or an atomic group containing an azide group can be introduced via the aforementioned linker structure. Examples of the azide group-introducing reagent to be used include silyl azide, azide phosphate, alkyl ammonium azide, inorganic azide, sulfonyl azide, PEG azide, and the like.

When an atomic group containing TCO is introduced as the second atomic group, TCO is directly introduced into the N-terminal or C-terminal of a peptide by using a commercially available click chemistry reagent containing TCO according to a conventional method, or an atomic group containing TCO can be introduced via the aforementioned linker structure.

The method for binding an antibody-modification peptide to an anti-CD20 antibody to obtain a peptide-modified antibody can be performed, for example, by dispersing the aforementioned antibody-modification peptide, an anti-CD20 antibody, a crosslinking agent, and a catalyst as necessary in an appropriate buffer, according to the description of WO 2017/217347. As the crosslinking agent, those mentioned above can be used. In addition, when binding the antibody-modification peptide and the anti-CD20 antibody, where necessary, buffer replacement including treating the solution containing the anti-CD20 antibody with an ultrafiltration filter, etc., and dispersing same in a buffer may be performed once or twice before binding with the antibody-modification peptide.

In one embodiment, the present disclosure relates to a method for producing a conjugate of an antibody-modification peptide and an anti-CD20 antibody, comprising a step of mixing an antibody-modification peptide modified with a crosslinking agent and an anti-CD20 antibody. By this step, a crosslinking reaction can occur between the antibody-modification peptide modified with the crosslinking agent and the anti-CD20 antibody. For example, in rituximab, the crosslinking reaction may occur site-specifically between the above-mentioned $Xaa_2$ amino acid residue of the antibody-modification peptide and Lys250 residue or Lys252 residue, preferably Lys252 residue, according to Eu numbering in human IgG Fc.

The conditions of the mixing step are not particularly limited as long as a crosslinking reaction occurs between the antibody-modification peptide and the anti-CD20 antibody. For example, the reaction can be performed by mixing an antibody-modification peptide and an anti-CD20 antibody in an appropriate buffer at room temperature (e.g., about 15°C to 30°C). The mixing step may be performed by adding as necessary an appropriate amount of a catalyst that promotes the crosslinking reaction.

In one embodiment, a solvent containing at least water is added to dissolve the anti-CD20 antibody. The solvent other than water includes, for example, dimethyl sulfoxide, acetonitrile, saline, and buffers such as sodium acetate buffer, ammonium acetate buffer, phosphate buffer, phosphate buffered saline, Tris buffer, HEPES buffer, tetramethylammonium acetate buffer, histidine buffer, and the like. When a buffer is used, the pH at 25°C is preferably set to 4.0 or more and 10.0 or less, more preferably 5.5 or more and 8.5 or less, from the aspect of the stability of the antibody. At the start of the crosslinking reaction, the concentration of the antibody is preferably set to 1.0 μmol/L or more as the lower limit and 1000 μmol/L or less, more preferably 500 μmol/L or less, as the upper limit.

Then, an antibody-modification peptide modified with a crosslinking agent and, where necessary, a catalyst are added and the mixture is dispersed at 10°C or higher and 30°C or lower.

The mixing ratio of the antibody-modification peptide and the anti-CD20 antibody in the mixing step is not particularly limited. The molar ratio of the antibody-modification peptide to the anti-EGFR antibody can be set to, for example, 1:1 to 20:1, preferably 2:1 to 20:1 or 5:1 to 10:1.

In a preferred embodiment, the molar ratio of the antibody-modification peptide to the anti-CD20 antibody in the above-mentioned mixing step can be 0.5 to 2.2, preferably 0.8 to 1.8. In this way, an antibody in which one molecule of antibody-modification peptide is bound to one molecule of anti-CD20 antibody (hereinafter referred to as "monovalent antibody") can be obtained efficiently.

The mixing time (reaction time) in the mixing step is not particularly limited as long as a crosslinking reaction occurs between the antibody-modification peptide and the anti-CD20 antibody. It is, for example, 1 min to 5 hr, preferably 10 min to 2 hr.

The peptide-modification antibody obtained through the above steps is a mixture containing an antibody in which one molecule of antibody-modification peptide is bound to one molecule of anti-CD20 antibody (hereinafter referred to as "monovalent antibody") and an antibody in which two molecules of antibody-modification peptide are bound to one molecule of anti-CD20 antibody (hereinafter referred to as "divalent antibody") at any ratio. This may be used as it is for the subsequent steps, or an unmodified antibody, a monovalent antibody, and a divalent antibody are separated and purified by a method such as filtration filter, membrane filter, column filled with various fillers, various chromatographies and the like, and only the antibody having any valence may be subjected to the subsequent steps. When the unmodified antibody cannot be separated from the antibody having other valence as a result of purification, a mixture containing these may be subjected to the subsequent steps.

When an unmodified antibody, a monovalent antibody, and a divalent antibody are separated and purified, any of the above-mentioned purification methods may be used for separation and purification. It is possible to use a column packed with various fillers. For example, a column packed with a filler in which a protein such as protein A, protein G, or the aforementioned antibody-modification peptide is bound to a carrier can be used. The shape of the carrier of the filler packed in such a column includes gel (e.g., column gel), particle, bead, nanoparticle, microparticle, macrobead, and the like. The materials of the carrier include magnetic substance, latex, agarose, glass, cellulose, sepharose, nitrocellulose, polystyrene, and other polymeric materials. Specific example is an IgG-BP column in which the aforementioned antibody-modification peptide is bound to a column gel (see WO 2021/080008).

Using the IgG-BP column and utilizing the difference in the interaction with respective antibody-modification peptides, the first antibody composition containing relatively large amounts of the unmodified antibody and the monovalent antibody and the second antibody composition containing a relatively large amount of the divalent antibody can be respectively separated and purified. The first antibody composition or the second antibody composition separated and purified may be used as it is for the click reaction in the subsequent step (B), or may be used for the click reaction in step (B) after adjusting the protein concentration of the peptide-modified antibody contained.

The click reaction in step (B) is performed between the first atomic group capable of click reaction which is contained in the chelating agent, and the second atomic group capable of click reaction which is contained in the peptide-modified antibody. By such click reaction, a binding group (substituent capable of conjugating with antibody) that links a chelating agent and an antibody is formed.

When the peptide-modified antibody and the complex obtained in step (A) are capable of click reaction, the

order of addition of these does not matter. For example, one of the complex and the peptide-modified antibody is added to a reaction container containing a solvent, and then the other is added to perform the reaction, or one of the chelating agent and the antibody is dispersed in a solvent and the other is added to the dispersion to perform the reaction. Alternatively, these may be simultaneously added to a reaction container containing a solvent to perform the reaction.

As the solvent to be used for the click reaction in step (B), a solvent containing water can be used. For example, water, saline, buffers such as sodium acetate buffer, ammonium acetate buffer, phosphate buffer, phosphate buffered saline, Tris buffer, HEPES buffer, tetramethylammonium acetate buffer and the like, and the like can be used. When a buffer is used, to simultaneously achieve the stability of the complex and the antibody, and the bond efficiency of these, the pH at 25°C is preferably set to not less than 4.0 and not more than 10.0, further preferably not less than 5.5 and not more than 8.5.

While the amount of the reaction mixture is not particularly limited, from the aspect of practicality in the production step, the lower limit at the start of step (B) is preferably not less than 0.001 mL, more preferably not less than 0.01 mL, further preferably not less than 0.1 mL, further more preferably not less than 1 mL, and the upper limit is preferably not more than 1000 mL, more preferably not more than 100 mL, further preferably not more than 10 mL, further more preferably not more than 1 mL. For example, the range of not less than 0.001 mL and not more than 1000 mL is preferable, and the range of not less than 0.1 mL and not more than 10 mL is more preferable.

As the concentrations of the chelating agent and the antibody in the reaction mixture, each independently, the lower limit at the start of step (B) is preferably not less than 0.001 μmol/L, more preferably not less than 0.01 μmol/L, further preferably not less than 0.1 μmol/L, further more preferably not less than 1.0 μmol/L, and the upper limit is preferably not more than 1000 μmol/L, more preferably not more than 100 μmol/L. For example, the range of not less than 0.1 μmol/L and not more than 1000 μmol/L is preferable, and the range of not less than 1 μmol/L and not more than 100 μmol/L is more preferable, from the aspect of the yield of the desired conjugate.

To prevent unintended denaturation of the antibody and increase the reaction efficiency, the upper limit of the reaction temperature of the click reaction in step (B) is preferably not more than 50°C, more preferably not more than 40°C. The lower limit of the reaction temperature is not particularly limited as long as the reaction proceeds, and is preferably not less than 15°C. The reaction time of the click reaction is, on the condition that it is the aforementioned reaction temperature, preferably not less than 5 min, more preferably not less than 10 min, preferably not more than 24 hr, more preferably not more than 20 hr. For example, the range of not less than 5 min and not more than 24 hr is preferable, and the range of not less than 10 min and not more than 20 hr is more preferable.

The obtained conjugate may be used as it is or purified using a filtration filter, a membrane filter, a column filled with various fillers, chromatography or the like.

In the conjugate produced by steps (A) and (B), the lysine residue in the Fc region of anti-CD20 antibody is specifically modified with a chelating agent. This conjugate comprises one or two molecules of the aforementioned chelating agent per one molecule of the antibody. The chelating agent site-specifically modifies the Fc region of the antibody of the present invention via a linker (L). The linker (L) is constituted of a chelate linker ($L_2$) that connects to a chelating agent, a first atomic group that connects to the linker ($L_2$), a second atomic group that can perform click reaction with the first atomic group, and an antibody-modification linker ($L_1$) that connects to the second atomic group (including an antibody-modification peptide represented by the above-mentioned formula (i)). Therefore, the linker (L) has a chemical structure derived from the first atomic group and the second atomic group. As such chemical structure, a triazole skeleton-containing structure represented by the aforementioned formula (10a) or (10b) or a pyridazine skeleton-containing structure represented by the aforementioned formula (10c) can be considered. Since the formula (10a) and the formula (10b) are isomers, they may be contained at any ratio.

(1-7) Radiopharmaceutical (Radiopharmaceutical (1))

A radiopharmaceutical refers to a composition that contains the radioconjugate of the present invention and is in a form suitable for in vivo administration to a subject. The radiopharmaceutical may be, for example, a radioconjugate produced by the method shown in the aforementioned (1-6) as it is, or can be produced by purifying same and dissolving same in a solvent mainly containing water and isotonic with the living body. In this case, the radiopharmaceutical is preferably in the form of an aqueous solution, and may contain other pharmaceutically acceptable components as necessary. An effective amount of the radiopharmaceutical is orally or parenterally, for example, intravenously, subcutaneously, intraperitoneally, intramuscularly, or the like, administered to a living body, and is used for treatment of cancer, diagnosis of cancer, detection of a lesion, or the like.

As used herein, the subject of administration is a human, or an animal such as mouse, rat, monkey, guinea pig, chimpanzee, sheep, goat, dog, cat, swine, bovine, horse or the like, but is not particularly limited. Preferred is a human.

A preferred target disease is a disease that overexpresses CD20. Specifically, suppression of CD20-positive B-cell non-hodykin lymphoma, CD20-positive B-cell lymphoproliferative disease under immunosuppressive conditions, granulomatosis with polyangiitis (Wegena's granulomatosis), microscopic polyangiitis, intractable nephrotic syndrome,

antibody-related rejection in ABO blood type incompatible transplants during kidney and liver transplantations, and the like can be mentioned.

As used herein, the "effective amount" is an amount that can afford useful diagnostic or therapeutic effects in a subject of administration. The effective amount to be administered to a subject varies depending on the type of subject, body weight of the subject, dosage form (tablet, injection, etc.) and route (oral administration, parenteral administration, etc.) of administration, severity of disease (e.g., cancer), and the like. Physicians and veterinarians can consider these factors and determine the appropriate effective amount.

The radiopharmaceutical of the present invention when stored at room temperature has a radiochemical purity of a certain level or above at the time of expiry of a period that is a multiple of not less than 1 and not more than 5 of the half-life, based on the half-life of the metal radionuclide constituting the radiopharmaceutical. When the above-mentioned metal radionuclide is a β-ray nuclide (e.g., Lu-177 or Y-90), the radiochemical purity of the conjugate is preferably not less than 90%, when stored at room temperature for 7 days after production. When the metal radionuclide is an α-ray nuclide (e.g., Ac-225), the radiochemical purity of the conjugate after storage for 14 days at room temperature from the completion of the production is preferably not less than 90%, more preferably not less than 95%, further preferably not less than 97%. The "room temperature" in the present specification preferably refers to the "ordinary temperature" defined in the Japanese Pharmacopoeia, which is specifically 15 to 25°C.

As used herein, the radiochemical purity refers to the percentage of the peak radioactivity (count) corresponding to the conjugate with respect to the total radioactivity (count) detected when the sample is analyzed with a commercially available radiation detector. High performance liquid chromatography and thin-layer chromatography can be used for the analysis of radiochemical purity, and thin-layer chromatography is preferably used. More preferably, thin-layer chromatography is used under the conditions described in the below-mentioned Examples.

As described above, the radiopharmaceutical of the present invention is preferably in the form of an aqueous solution. It is more preferably in the form of a buffer from the aspect of maintaining the radiochemical purity as described above. As the buffer, any buffer used in an antibody drug containing an anti-CD20 antibody or ADC of an anti-CD20 antibody as an active ingredient can be used. As a nonlimiting example, histidine buffer, phosphate buffer, acetate buffer, or citrate buffer can be used. The histidine buffer is composed of histidine and a salt thereof, for example, histidine and hydrochloride thereof or histidine and acetate thereof. The acetate buffer is composed of acetic acid and a salt thereof, for example, acetic acid and a sodium salt thereof. The phosphate buffer is composed of phosphoric acid and a salt thereof, for example, sodium hydrogen phosphate, potassium dihydrogen phosphate, and the like. The citrate buffer is composed of citric acid and a salt thereof and can be composed of, for example, citric acid and a sodium salt thereof. The radiopharmaceutical of the present invention may contain any sugar such as sucrose (refined white sugar), and may also contain a solubilizer such as polysorbate 20 and polysorbate 80, a metal sealing agent such as diethylenetriaminpentaacetic acid (DTPA) or a salt thereof, ethylenetriaminetetraacetic acid (edetic acid, EDTA) or a sodium salt thereof, and may contain human serum albumin.

The radiopharmaceutical of the present invention can be used for radionuclide therapy of cancer by selecting metal radionuclides that have a therapeutic effect, specifically nuclide that emits α rays or nuclide that emits β rays (preferably Ac-225, Y-90, Lu-177, more preferably Ac-225). In this radionuclide therapy, the radiopharmaceutical of the present invention is administered by intravenous injection or orally to cause accumulation of the radioconjugate of the present invention in a lesion site such as a cancer primary lesion or metastatic lesion, and cancer cells at the lesion site are destroyed by the radiation emitted from the metal radionuclide. The amount to be administered and dose of the radiopharmaceutical of the present invention is appropriately determined by the efficacy of the active ingredient, the mode and route of administration, the stage of cancer progression, the body type, body weight and age of the patient, and the type and amount of the therapeutic drug used in combination for other diseases.

In addition, by selecting a radionuclide that emits positron or a radionuclide that emits γ rays (preferably, Ga-68, Zr-89, In-111, more preferably Zr-89) as the metal radionuclide, it can be used for cancer diagnosis or lesion detection. A radiopharmaceutical using radionuclide that emits positron can be preferably used for PET (Positron Emission Tomography) examination, and a radiopharmaceutical using radionuclide that emits γ-rays can be preferably used for SPECT (Single Photon Emission Computed Tomography) examination. This may also be used in combination with cancer diagnosis or lesion detection in the aforementioned radionuclide therapy of cancer. The diagnostic radiopharmaceutical for cancer of the present invention may be used for diagnosis before performing radionuclide therapy for cancer, or may be used for diagnosis after performing radionuclide therapy for cancer. Using the radiopharmaceutical for diagnosis before conducting a radionuclide therapy for cancer, selection of treatment can be performed based on whether or not to perform radionuclide therapy for cancer using the radiopharmaceutical of the present invention provided with a metal nuclide that emits α-rays. Using the radiopharmaceutical for diagnosis after conducting a radionuclide therapy for cancer, moreover, whether or not radionuclide therapy for cancer using the radiopharmaceutical of the present invention is effective can be judged, and a treatment plan including an increase or a decrease of dose and the like can be optimized.

(2) Radiopharmaceutical (Radiopharmaceutical (2))

Another embodiment of the present invention is a radiopharmaceutical containing a conjugate of a chelating agent chelated with a metal radionuclide and an anti-CD20 antibody as an active ingredient, wherein the linkage between the anti-CD20 antibody and the chelating agent does not contain a thiourea bond, when stored at room temperature, it has a radiochemical purity of a certain level or above at the time of expiry of a period that is a multiple of not less than 1 and not more than 5 of the half-life, based on the half-life of the metal radionuclide constituting the radiopharmaceutical. When the above-mentioned metal radionuclide is a β-ray nuclide (e.g., Lu-177 or Y-90), the radiochemical purity of the aforementioned conjugate is preferably not less than 90%, when stored at room temperature for 7 days after production. Also, when the metal radionuclide is an α-ray nuclide (e.g., Ac-225), the radiochemical purity of the conjugate is preferably not less than 90%, more preferably not less than 95%, further preferably not less than 97%, when stored at room temperature for 14 days after production. The room temperature is as defined in the aforementioned radiopharmaceutical (1).

In the radiopharmaceutical (2), the following methods (a) to (d) can also be used in conjugating the chelating agent and the anti-CD20 antibody, in addition to the site-specific modification method using a peptide. Since other part is the same as in the radiopharmaceutical (1), the explanation is omitted.

(a) method for modifying a sulfhydryl (SH) group generated by partially reducing a disulfide bond (SS bond) between polypeptide chains at the hinge site of an antibody with a chelating agent or linker (L) having a maleimide group reactive with the SH group
(b) method for modifying cysteine newly introduced into an antibody by an amino acid mutation by genetic engineering with a chelating agent or linker (L) having a maleimide group
(c) method for modifying an azide group of azidized lysine newly introduced into an antibody by an amino acid mutation by genetic engineering with a chelating agent or linker (L) having alkyne (e.g., Dibenzylcyclooctyne: DBCO) by using a click reaction
(d) method for modifying glutamine introduced into a specific position of an antibody with a chelating agent or linker (L) having a side chain of lysine by using transglutaminase

In the present invention, a peptide that site-specifically modifies an anti-CD20 antibody and a chelating agent are linked without using a thiourea bond. Therefore, a radioconjugate and a radiopharmaceutical that are stable even at room temperature can be obtained. Since the site-specific modification of antibody can contain monovalent antibody or divalent antibody or both of these in any proportion, unlike random modification, radioconjugate and radiopharmaceutical with stable quality can be obtained. In addition, the radioconjugate of the present invention maintains efficacy equivalent to conventional one. Therefore, according to the present invention, an anti-CD20 antibody conjugate and a radiopharmaceutical thereof with higher quality while maintaining efficacy can be provided.

The following embodiments are also encompassed in the technical idea of the present invention.

[1] A conjugate of an anti-CD20 antibody and a chelating agent, wherein the aforementioned chelating agent is chelated with a metal radionuclide, the aforementioned anti-CD20 antibody and the chelating agent are linked by a linker (L), and the aforementioned linker (L) does not contain a thiourea bond.
[2] The conjugate of [1], which is a conjugate of an anti-CD20 antibody site-specifically modified with a peptide and a chelating agent, wherein the aforementioned peptide and the aforementioned chelating agent are linked by the aforementioned linker (L).
[3] The conjugate of [1], wherein the aforementioned chelating agent is DOTAGA (α-(2-Carboxyethyl)-1,4,7,10-tetraazacyclododecane-1,4,7,10-tetraacetic acid).
[4] The conjugate of [2] or [3], wherein the aforementioned peptide is an amino acid sequence consisting of 13 to 17 amino acid residues and is represented by the following formula (i):

$$(Xa)\text{-}Xaa_1\text{-}(Xb)\text{-}Xaa_2\text{-}(Xc)\text{-}Xaa_3\text{-}(Xd) \qquad (i)$$

in the formula (i), Xa, Xb, Xc and Xd are continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy $a+b+c+d \leq 14$,
$Xaa_1$ and $Xaa_3$ are each independently an amino acid residue derived from an amino acid having a thiol group in the side chain, or an amino acid residue derived from an amino acid having a haloacetyl group in the side chain, provided that one of $Xaa_1$ and $Xaa_3$ is an amino acid residue derived from an amino acid having a thiol

group in the side chain,

$Xaa_1$ and $Xaa_3$ are connected to form a ring structure, and

$Xaa_2$ is a lysine residue, an arginine residue, a cysteine residue, an aspartic acid residue, a glutamic acid residue, 2-aminosuberic acid, or diamino propionic acid, and modified with a crosslinking agent.

[5] The conjugate of any one of [1] to [4], wherein the aforementioned metal radionuclide is Ac-225, Y-90, Lu-177, or Zr-89.

[6] The conjugate of any one of [1] to [5], wherein the aforementioned linker (L) comprises the formula (10a), the formula (10b), or the formula (10c):

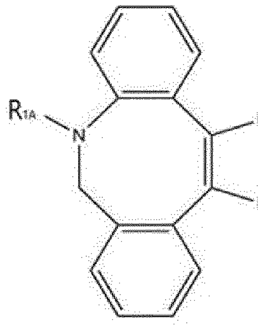

(1 0 a)

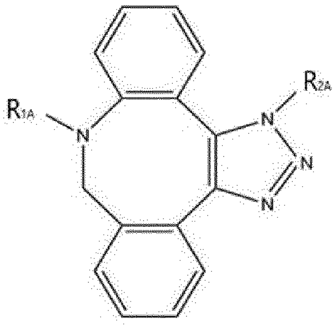

(1 0 b)

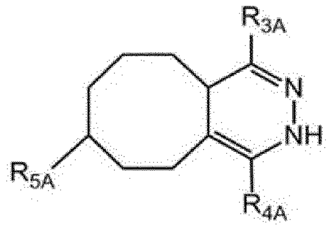

(1 0 c)

In the formula (10a) and the formula (10b), $R_{1A}$ is a binding site with a chelating agent, and $R_{2A}$ is a binding site with the aforementioned antibody or the aforementioned peptide. In the formula (10c), one of $R_{3A}$ and $R_{4A}$ is a hydrogen atom, a methyl group, a phenyl group or a pyridyl group, and the other is a binding site with the aforementioned chelating agent, and $R_{5A}$ is a binding site with the aforementioned antibody or the aforementioned peptide.

[7] The radioconjugate of [6], comprising a polyethylene glycol group between the linkage site with the aforementioned antibody or the aforementioned peptide and the aforementioned antibody or the aforementioned peptide.

[8] The radioconjugate of any one of [2] to [7], which is conjugated by a click reaction of an anti-CD20 antibody site-specifically modified with the aforementioned peptide having an azide group introduced into the N-terminal, and a radioactive metal complex of DOTAGA-DBCO represented by the following formula:

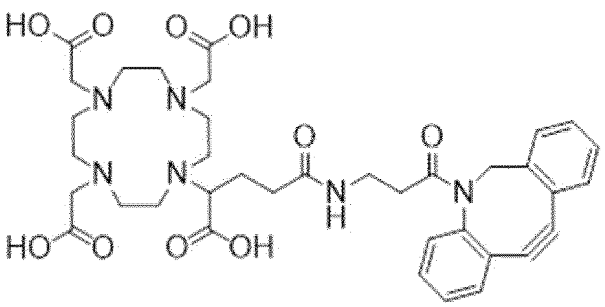

DOTAGA-DBCO

[9] The conjugate of any one of [1] to [8], wherein the aforementioned anti-CD20 antibody is rituximab.

[10] A radiopharmaceutical comprising the conjugate of any one of [1] to [9] as an active ingredient.

[11] The radiopharmaceutical of [10], which is used in a radionuclide therapy for cancer.

[12] The radiopharmaceutical of [10], which is used in cancer diagnosis.

[13] The radiopharmaceutical of [12], which is used in combination with the radionuclide therapy for cancer using the radiopharmaceutical in [11].

[14] A radiopharmaceutical comprising a conjugate of a chelating agent chelated with a metal radionuclide and an anti-CD20 antibody as an active ingredient, wherein the linkage between the anti-CD20 antibody and the chelating agent does not contain a thiourea bond, and the conjugate has a radiochemical purity of not less than 90% when stored at room temperature for 7 days.

[15] The radiopharmaceutical of [14], wherein the aforementioned conjugate is any one of [1] to [9].

[16] The radiopharmaceutical of [15], which is used in a radionuclide therapy for cancer.
[17] The radiopharmaceutical of [15], which is used in cancer diagnosis.
[18] The radiopharmaceutical of [17], which is used in combination with the radionuclide therapy for cancer using a radiopharmaceutical in [16].
[19] A radiopharmaceutical comprising a conjugate of a chelating agent chelated with a metal radionuclide and an anti-CD20 antibody as an active ingredient and satisfying the following condition (1) or (2), wherein the linkage between the anti-CD20 antibody and the chelating agent does not contain a thiourea bond:

(1) the aforementioned metal radionuclide is $^{177}Lu$ or $^{90}Y$, and the aforementioned conjugate has a radiochemical purity of not less than 90% when stored at room temperature for 7 days
(2) the aforementioned metal radionuclide is $^{225}Ac$, and the aforementioned conjugate has a radiochemical purity of not less than 90% when stored at room temperature for 14 days.

[20] A radiopharmaceutical comprising a conjugate of a chelating agent chelated with a metal radionuclide and an anti-CD20 antibody as an active ingredient, wherein the linkage between the anti-CD20 antibody and the chelating agent does not contain a thiourea bond, and the conjugate has a radiochemical purity of not less than 90% at the time of expiry of a period that is a multiple of not less than 1 and not more than 5 of the aforementioned half-life, based on the half-life of the aforementioned metal radionuclide.

[Example]

The present invention is described in more detail in the following by way of examples. However, the scope of the present invention is not limited by the examples. In the Tables below, the column with "-" indicates no performance.

[Example 1] Production of conjugate with rituximab by using $^{225}Ac$-labeled DOTAGA-DBCO

(1. Antibody modification step)

A peptide (SEQ ID NO: 17) containing 17 amino acid residues represented by the following formula (P3) was obtained by the method described in WO 2017/217347. The amino acid sequence of this peptide was the same as the sequence in which $Xaa_2$ of SEQ ID NO: (2) was a lysine residue, and the side chain terminal amino group of the lysine residue was modified with the structure shown by $R_1$. In addition, two cysteine residues form a disulfide bond with each other, and to the N-terminal of the peptide was added ethyl azide as an atomic group containing an azide group, which is the second atomic group, via a linker ($L_1$) structure having diglycolic acid and eight PEGs.

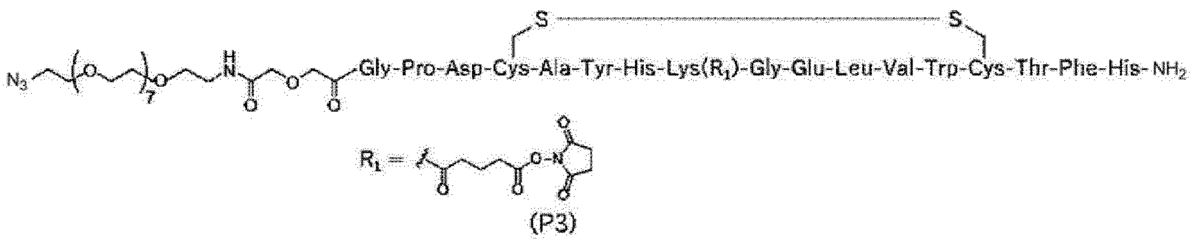

(P3)

In the formula (P3), Gly is glycine, Pro is proline, Asp is aspartic acid, Cys is cysteine, Ala is alanine, Tyr is tyrosine, His is histidine, Lys is lysine, Glu is glutamic acid, Leu is leucine, Val is valine, Trp is tryptophan, Thr is threonine, and Phe is phenylalanine.

A mixture of the aforementioned peptide and rituximab (manufactured by Merck) in a 0.2 mol/L acetic acid·sodium acetate buffer (pH 6.0) was reacted at room temperature for 60 min to give a solution containing a peptide-modified antibody. The peptide-modified antibody has an Fc region of the antibody site-specifically modified by the above-mentioned peptide.

Then, the solution was passed through the IgG-BP column to obtain the first antibody composition containing relatively large amounts of the unlabeled antibody and monovalent antibody. The concentration of the monovalent antibody contained in the recovered fraction was adjusted with 0.025 mol/L sodium citrate buffer (pH 5.5) containing 0.154 mol/L sodium chloride such that the concentration was 15 mg/mL. An obtained solution containing the first antibody composition was subjected to the below-mentioned labeling step.

(2. Complex formation step)

DOTAGA-DBCO represented by the following formula was produced based on the method described in Bernhard et al. DOTAGA-Anhydride: A Valuable Building Block for the Preparation of DOTA-Like Chelating Agents Chem. Eur. J. 2012, 18, 7834-7841. This chelating agent was dispersed in 0.1 mol/L sodium acetate buffer (pH 6.0) as a solvent to give a dispersion containing 1.7 mmol/L chelating agent. A reaction mixture of the dispersion (0.003 mL), 0.1 mol/L sodium acetate buffer (pH 6.0, 0.045 mL), 0.1 mol/L hydrochloric acid (0.001 mL), and $^{225}Ac$ ion-containing solution (0.2 mol/L aqueous hydrochloric acid solution, radioactivity concentration 994 MBq/mL, prepared from one produced by Oak Ridge National Laboratory, liquid amount 0.002 mL) about 2.0 MBq (calculated by attenuation from the level of radioactivity at test date and time) as a radioactive metal source was reacted under heating conditions to give a $^{225}Ac$ complex solution. The molar ratio of the chelating agent and the radioactive metal ion was chelating agent:$^{225}Ac$ ion = about 1230:1, and the heating temperature of the reaction mixture was set to 70°C, and the heating time was set to 30 min.

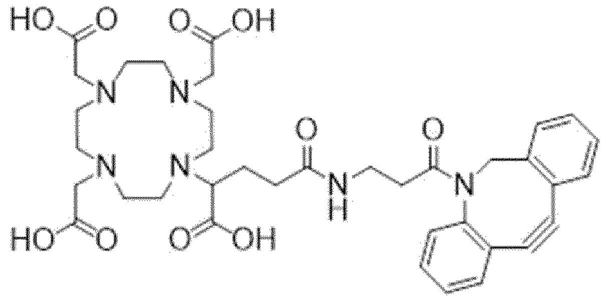

DOTAGA-DBCO

The radiochemical purity (RCP) of the obtained $^{225}Ac$ complex was measured by the following method. That is, a part of the $^{225}Ac$ complex solution was developed by thin layer chromatography (manufactured by Agilent, model number: SGI0001, eluent: acetonitrile/water mixed solution (volume ratio 1:1)), and then measured by radio γ-TLC Analyzer (manufactured by raytest, MODEL GITA Star). The percentage of the radioactivity (count) of the peak detected near the solvent front with respect to the detected total radioactivity (count) was defined as the RCP (%) of the $^{225}Ac$ complex. As a result, the RCP of the $^{225}Ac$ complex was 93%. The obtained $^{225}Ac$ complex solution was directly used for the labeling step.

(3. Labeling step)

To a solution of the unpurified $^{225}Ac$ complex obtained via the aforementioned step (2. Complex formation step) was added a solution containing a peptide-modified antibody (monovalent antibody) obtained in the above-mentioned step (1. Antibody modification step), and a click reaction was performed at 37°C for 120 min to give $^{225}Ac$ complex-labeled antibody. The amount of the $^{225}Ac$ complex and the amount of the peptide-modified antibody (monovalent antibody) were 5.1 nmol and 6.0 nmol, respectively, and the molar ratio of the DBCO group and the azide group was about 1:1.2. The reaction rate (%) of the unpurified $^{225}Ac$ complex-labeled antibody was 75%. Here, the reaction rate (%) means the radiochemical purity (%) of the $^{225}Ac$ complex-labeled antibody with respect to the labeling rate (%) in the complex formation step, and the labeling rate (%) means the proportion (%) of the radioactivity of the $^{225}Ac$ complex with respect to the charged radioactivity level.

Furthermore, a solution of the $^{225}Ac$ complex-labeled antibody obtained by reacting at 37°C for 2 hr was purified using ultrafiltration filter (manufactured by Merck, model number: UFC505096). The RCP of the $^{225}Ac$ complex-labeled antibody after purification was 93% and the radiochemical yield (RCY) thereof was 61%.

The measurement method of the RCP and RCY of the $^{225}Ac$ complex-labeled antibody was as follows. That is, thin layer chromatography (manufactured by Agilent, model number: SGI0001, developing solvent was developed with a solution of acetonitrile:0.1 mmol/L EDTA solution (volume ratio 1:1)) and thereafter measured by radio γ-TLC Analyzer (manufactured by raytest, MODEL GITA Star). The percentage of the radioactivity (count) of the peak detected near the origin to the total radioactivity (count) detected was defined as the RCP (%). In addition, the percentage of the radioactivity (radioactivity level calculated from the count measured by γ ray spectrometer (Ge semiconductor detector: GMX10P4-70 (manufactured by ORTEC), Multi Channel Analyzer: M7-000 (manufactured by SEIKO EG&G), data processing: Spectrum Navigator:DS-P300 (manufactured by SEIKO EG&G) and Gamma Studio:DS-P600 (manufactured by SEIKO EG&G)) recovered after ultrafiltration purification with respect to the total radioactivity (similar to the above, the radioactivity level calculated from the count measured by γ ray spectrometer) added at the start of the labeling step

was defined as the RCY (%).

[Comparative Example 1] Production of conjugate with rituximab by using $^{225}$Ac-labeled DOTA-DBCO

The operation was performed according to Example 1 except that DOTAGA-DBCO was changed to the following DOTA-DBCO. As a result, the reaction rate (%) was 71% at 2 hr after the reaction, and RCP of the $^{225}$Ac complex-labeled antibody after purification was 99% and RCY thereof was 46%.

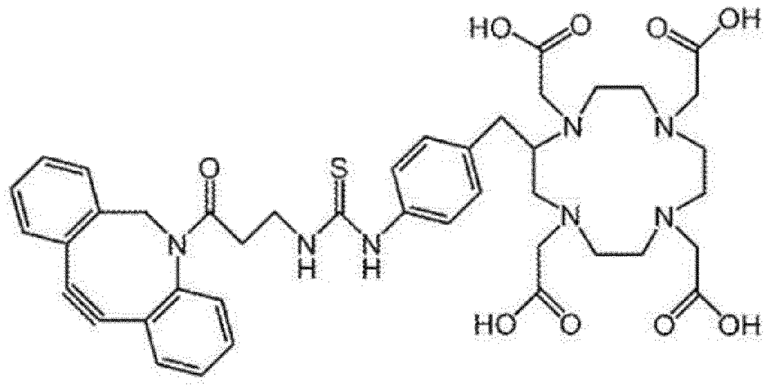

DOTA-DBCO

[Example 2] Formulation step

A portion of each of the radioconjugates prepared as described in Example 1 and Comparative Example 1 was placed in a 0.5 mL Eppendorf tube (LoBind, manufactured by Eppendorf) and diluted with a storage buffer (mixed solution of 0.025 mol/L sodium citrate buffer (pH 5.5) containing 0.154 mol/L sodium chloride and 0.025 mol/L sodium citrate buffer (pH 5.5) containing 0.07 weight/ volume % polysorbate 80 and 0.154 mol/L sodium chloride).

[Evaluation 1] Stability evaluation

Each radioconjugate obtained in Example 2 was stored at room temperature, and radiochemical purity and antigen binding activity were evaluated at each time point (0 day point, 1 day point, 7 day point, 14 day point, 21 day point, 30 day point, and/or 40 day point). When the metal radionuclide is Ac-225, 14 days after the end of production corresponds to about 1.5 half-life, 21 days after the end of production corresponds to about 2 half-life, 30 days after the end of production corresponds to about 3 half-life, and 40 days after the end of production corresponds to about 4 half-life.

[Evaluation 1-1] (Radiochemical purity (RCP)

RCP was analyzed by thin layer chromatography (TLC). The TLC conditions were similar to those used for examining the reaction rate in Example 1. The results are shown in Table 1.

[Table 1]

| | radiochemical purity (%) | | | | | | |
|---|---|---|---|---|---|---|---|
| | 0 day point | 1 day point | 7 day point | 14 day point | 21 day point | 30 day point | 40 day point |
| radioconjugate (Example 1) | 99.9 | 99.9 | 99.3 | 99.5 | 94.9 | 93.1 | 89.6 |
| radioconjugate (Comparative Example 1) | 99.9 | 99.4 | 79.1 | 84.1 | 68.2 | 67.6 | 64.8 |

The radioconjugate produced as described in Example 1 containing no thiourea bond maintained an RCP of 99% or more when stored at room temperature for 7 days after completion of the production. It maintained an RCP of 99% or more when stored at room temperature for 14 days after completion of the production. Also, it maintained an

RCP of 90% or more even when stored at room temperature for 30 days after completion of the production.

The radioconjugate produced as described in Comparative Example 1 containing a thiourea bond maintained an RCP of 75% or more but below 99% when stored at room temperature for 7 days after completion of the production. When stored at room temperature for 14 days after completion of the production, the RCP was less than 85%.

[Evaluation 1-2] Antigen binding activity

Antigen binding activity was confirmed by in vitro autoradiography (ARG) (production day (0 day point) alone). Ramos cells of a CD20-positive human Burkitt lymphoma-derived cell line purchased from ATCC (American Type Culture Collection), and CCRF-CEM cells of a CD20-negative human acute lymphoblastic leukemia/T lymphocyte-like cell line were administered subcutaneously to the flanks of female BALB/c nu/nu mice (manufactured by Charles River Laboratories Japan, Inc.) at $5\times10^6$ cells to prepare tumor-bearing mice. Thereafter, Ramos tumor and CCRF-CEM tumor were excised and embedded in Tissue-Tek O.C.T. Compound (Sakura Finetek Japan Co., Ltd.) to prepare frozen sections. The radioconjugates obtained in Example 1 and Comparative Example 1 were added to 1% bovine serum albumin-containing PBS each at 1 kBq/mL, and Ramos tumor section and CCRF-CEM tumor section were immersed therein. After contacting the sections with the imaging plate, they were read with a scanner-type image analyzer (Typhoon FLA 7000 manufactured by GE Healthcare Japan) to evaluate the level of radioactivity bound to the sections. The results are shown in Fig. 3. The binding activity to CD20 was confirmed in all of the radioconjugates prepared as described in Example 1 and Comparative Example 1. Both radioconjugates prepared as described in Example 1 and Comparative Example 1 bound to the Ramos tumor section alone, demonstrating CD20 selective binding. The radioactivity bound to Ramos tumor section was higher in the samples using the radioconjugates prepared as described in Example 1 than in the radioconjugates prepared as described in Comparative Example 1.

[Evaluation 1-3] Stability evaluation of $^{225}$Ac complex-labeled rituximab in plasma

Radioconjugates produced as described in Example 1 were stored at 37°C for 2 weeks in the plasma of each animal species (human (human plasma (pool, heparin), manufactured by Cosmo Bio Company, Limited), mouse (mouse plasma pool BALB/c-nu lineage, manufactured by Charles River Laboratories Japan, Inc.), rat (plasma (pool) Wistar, manufactured by Charles River Laboratories Japan, Inc.), monkey (Macaca fascicularis plasma, manufactured by Charles River Laboratories Japan, Inc.)), and the stability of $^{225}$Ac-labeled rituximab in the plasma was evaluated at each time point (0 day point, 1 day point, 7 day point, 14 day point (human plasma alone)). Plasma of each animal species was incubated at 37°C, and a radioconjugate produced as described in Example 1 was added at a final concentration of 100 kBq/mL. At each evaluation time point, immunoprecipitation was performed using Dynabeads (registered trademark) Protein G (manufactured by Thermo Fisher Scientific), and after washing with PBS containing 0.1% Tween 20, antibodies bound to Protein G were collected. The PBS containing 0.1% Tween 20 used for washing was collected, and the radioactivity of the collected washing solution and the radioactivity of the collected antibody were measured using an Autowell gamma counter (2480 WIZARD$^2$ gamma counter, manufactured by PerkinElmer). The ratio of the radioactivity of the recovered antibody to the total of the radioactivity of PBS containing 0.1% Tween 20 used for washing the beads and the radioactivity of the recovered antibody was calculated, whereby the percentage of $^{225}$Ac-labeled rituximab bound to Protein G was evaluated. The results thereof are shown in Table 2. The values in the Table indicate mean±standard deviation (n=3) of the ratio of the radioactivity of the recovered antibody to the total of the radioactivity of PBS containing 0.1% Tween 20 used for washing the beads and the radioactivity of the recovered antibody.

[Table 2]

| | recovery rate (%) by beads | | | |
|---|---|---|---|---|
| | 0 day point | 1 day point | 7 day point | 14 day point |
| human plasma | 95.0±2.8 | 90.5±2.4 | 81.0±4.1 | 76.4±0.8 |
| monkey plasma | 92.9±1.2 | 90.4±0.5 | 90.5±0.8 | - |
| mouse plasma | 98.1±0.4 | 95.0±1.3 | 93.1±0.4 | - |
| rat plasma | 97.4±0.1 | 94.8±1.8 | 90.4±5.1 | - |

The radioconjugate produced as described in Example 1 showed a recovery rate using beads of 80% or more when stored for 7 days in the plasma of each animal species, and maintained a recovery rate using beads of 76% or more even when stored for 14 days in human plasma, and is considered to be stable in the plasma of each animal

evaluated.

[Evaluation 2] Efficacy evaluation

A subcutaneous tumor-bearing model of Ramos cells was prepared using mice, and the antitumor effect of the radioconjugate produced as described in Example 1 and Comparative Example 1 was confirmed.

Ramos cells purchased from ATCC were suspended in RPMI1640 medium (gibco, manufactured by Thermo Fisher Scientific) and administered subcutaneously to the flanks of female 5-week-old BALB/c nu/nu (Charles River Laboratories Japan, Inc.) at $5\times10^6$ cells to prepare tumor-bearing mice. The tumor volume was allowed to grow to 100 to 400 $mm^3$, and individuals with a shape suitable for tumor diameter measurement were randomly grouped. The tumor volume and body weight of each mouse at that time are shown in Table 3. The tumor volume was calculated according to the following formula.

$$\text{tumor volume } (mm^3) = (\text{tumor major axis} \times (\text{tumor minor axis})^2) \times 1/2$$

[Table 3]

| | tumor volume mean±standard deviation ($mm^3$) | body weight mean±standard deviation (g) |
|---|---|---|
| radioconjugate (Example 1) group | 217.2±94.3 | 20.7±1.4 |
| radioconjugate (Comparative Example 1) group | 221.3±108.2 | 20.1±1.2 |
| antibody control group | 236.9±97.1 | 19.7±2.3 |
| Vehicle group | 242.8±91.9 | 20.7±1.2 |

The radioconjugates prepared as described in Example 1 and Comparative Example 1 were administered into the tail vein at a dose of 15 kBq/mouse (56 μg/mouse as rituximab). As a control group, a group administered with rituximab with the same amount of antibody as each radioconjugate (antibody control group) and a Vehicle group administered with a storage buffer were set. Each group contained 6 mice, and the tumor volume was measured over time up to 20 days after administration. The changes in tumor volume over time are shown in Fig. 4.

The groups administered with the radioconjugates prepared as described in Example 1 and Comparative Example 1 showed a significant difference in the antitumor effect as compared with the two control groups (antibody control group and Vehicle group) at 20 day point after the administration ($P<0.05$). No significant difference in antitumor effect was observed between the group administered with the radioconjugate prepared as described in Example 1 and the group administered with the radioconjugate prepared as described in Comparative Example 1. For determination of significant difference, Steel-Dwass test was performed using statistical analysis software Stat Preclinica (manufactured by Takumi Information Technology Inc.).

[Example 3] Production of conjugate with rituximab by using $^{89}$Zr-labeled DOTAGA-DBCO

(1. Complex formation step)

DOTAGA-DBCO was dispersed in 0.156 mol/L sodium acetate buffer (pH 5.5) as a solvent to give a dispersion containing 0.3 mmol/L chelating agent. A reaction mixture of the dispersion (0.045 mL), 0.156 mol/L sodium acetate buffer (pH 5.5, 0.030 mL) containing 0.15 mol/L gentinic acid, and $^{89}$Zr ion-containing solution (0.1 mol/L aqueous hydrochloric acid solution, radioactivity concentration 1.9 GBq/mL, prepared from one manufactured by Nihon Medi-Physics Co., Ltd., liquid amount 0.03 mL), 57.2 MBq, as a radioactive metal source was reacted under heating conditions to give a $^{89}$Zr complex solution. The molar ratio of the chelating agent and the radioactive metal ion was chelating agent:$^{89}$Zr ion = about 630:1, and the heating temperature of the reaction mixture was set to 70°C, and the heating time was set to 60 min.

The RCP of the obtained $^{89}$Zr complex was measured by the following method. That is, a part of the $^{89}$Zr complex solution was developed by thin layer chromatography (manufactured by Agilent, model number: SGI0001, developing solvent: acetonitrile/water mixed solution (volume ratio 1:1)), and then measured by radio γ-TLC Analyzer

(manufactured by raytest, MODEL GITA Star PS). The percentage of the radioactivity (count) of the peak detected near the solvent front with respect to the detected total radioactivity (count) was defined as the RCP (%) of the $^{89}Zr$ complex. As a result, the RCP of the $^{89}Zr$ complex was 98%. The obtained $^{89}Zr$ complex solution was used as it was in the labeling step.

(2. Labeling step)

A solution of the unpurified $^{89}Zr$ complex obtained in the aforementioned step (1), and a solution containing a peptidemodified antibody (monovalent antibody) obtained in the same manner as in Example 1 were mixed without purification, and a click reaction was performed at 37°C for 2 hr to give a $^{89}Zr$ complex-labeled antibody. The molar ratio of DBCO and azide was each about 1:1. The reaction rate (%) of the unpurified $^{89}Zr$ complex-labeled antibody of the Example was 72%. Here, the reaction rate (%) means RCP (%) of the $^{89}Zr$ complex-labeled antibody with respect to the labeling rate (%) in the complex formation step, and the labeling rate (%) means the proportion (%) of the amount of radioactivity (%) of the $^{89}Zr$ complex with respect to the charged radioactivity amount.
Furthermore, a solution of the $^{89}Zr$ complex-labeled antibody obtained by reacting at 37°C for 2 hr was purified using ultrafiltration filter (manufactured by Merck, model number: UFC803096). The radiochemical purity (RCP) of the $^{89}Zr$ complex-labeled antibody after purification was 96% and the radiochemical yield (RCY) thereof was 49%.
The measurement of the RCP and RCY of the $^{89}Zr$ complex-labeled antibody was performed in the same manner as in Example 1.

[Comparative Example 2] Production of conjugate with rituximab by using $^{89}Zr$-labeled DOTA-DBCO

The operation was performed according to Example 3 except that DOTAGA-DBCO was changed to DOTA-DBCO. As a result, the reaction rate (%) was 61% at 2 hr after the reaction, and the radiochemical purity (RCP) of the $^{89}Zr$ complex-labeled antibody after purification was 93% and the radiochemical yield (RCY) thereof was 48%.

[Example 4] Formulation step

A portion of each of the radioconjugates prepared as described in Example 3 and Comparative Example 2 was placed in a 5 mL Eppendorf tube (LoBind, manufactured by Eppendorf) and diluted with a storage buffer (mixed solution of 0.025 mol/L sodium citrate buffer (pH 5.5) containing 0.154 mol/L sodium chloride, and 0.025 mol/L sodium citrate buffer (pH 5.5) containing 0.07 weight/volume % polysorbate 80 and 0.154 mol/L sodium chloride).

[Evaluation 3-1] Antigen binding activity of $^{89}Zr$ complex-labeled rituximab

The antigen binding activity was confirmed by in vitro ARG as in Evaluation 1-2. By performing the same evaluation of a solution obtained by adding rituximab to the solution of Example 3, the specificity of each radioconjugate for CD20 was confirmed. The results are shown in Fig. 5. The binding activity to CD20 was confirmed in all of the radioconjugates prepared as described in Example 3 and Comparative Example 2. The radioconjugates prepared as described in Example 3 and Comparative Example 2 bound to the Ramos tumor section alone, demonstrating CD20 selective binding. In the solution added with rituximab, binding to the Ramos tumor section was inhibited, and CD20 specificity of binding was confirmed.

[Evaluation 4-1] Evaluation of tumor accumulation of $^{89}Zr$ complex-labeled rituximab

Subcutaneous tumor-bearing models of Ramos cells and CCRF-CEM cells were prepared using mice, and the tumor accumulation of the radioconjugates prepared as described in Example 3 was confirmed.
Ramos cell and CCRF-CEM cell tumor-bearing mice were prepared in the same manner as in Evaluation 2. About two weeks after the tumor-bearing treatment, it was confirmed that the tumor volume was approximately 100 to 400 $mm^3$. A radioconjugate produced as described in Example 3 was administered at a dose of 4 MBq/mouse (each group n=3) into the tail vein. The tumor volume and body weight of each mouse at that time are shown in Table 4. The tumor volume was calculated according to the following formula.

$$\text{tumor volume } (mm^3) = (\text{tumor major axis} \times (\text{tumor minor axis})^2) \times 1/2$$

[Table 4]

| | tumor volume mean±standard deviation ($mm^3$) | body weight mean±standard deviation (g) |
|---|---|---|
| Ramos cell tumor-bearing group | 206.1±191.9 | 21.4±0.9 |
| CCRF-CEM cell tumor-bearing group | 297.2±30.3 | 20.3±0.9 |

After 186 hr from the administration of the radioconjugates prepared as described in Example 3, the mice were euthanized by blood sampling from the heart under isoflurane anesthesia, and blood and tumors were collected. The weights of the collected blood and tumors were measured, and the counting rates of the blood and tumors were measured using a γ-ray well scintillation measuring device (JDC-1712, manufactured by Hitachi, Ltd.). Using the obtained tissue weight, counting rate, and administered radioactivity, the accumulation rate of administered radioactivity per unit weight was calculated. Using the calculated accumulation rate of administered radioactivity per unit weight, the accumulation ratio in the tumor relative to the blood was calculated, and the CD20 selectivity of the tumor accumulation of the radioconjugates prepared as described in Example 3 was evaluated. The results are shown in Fig. 6.

The accumulation ratio of tumor to blood was higher in CD20-positive Ramos tumor than in CD20-negative CCRF-CEM tumor. CD20-selective accumulation of the radioconjugate prepared as described in Example 3 was confirmed.

[Evaluation 5-1] In vitro efficacy comparison between $^{225}$Ac complex-labeled rituximab and existing drugs

The cytotoxic effect of the radioconjugate produced as described in Example 1 using cultured cells was compared with that of existing drugs. CD20-positive human diffuse large B-cell lymphoma-derived SU-DHL-2 cells purchased from ATCC were cultured in RPMI1640 medium (gibco, manufactured by Thermo Fisher Scientific), and a radioconjugate produced as described in Example 1 was diluted with each medium to 0, 0.001, 0.01, 0.03, 0.1, 0.3, 1, 3, 10, 30 kBq/mL (0.000874, 0.00874, 0.0262, 0.0874, 0.262, 0.874, 2.62, 8.74, 26.2 ug/mL as rituximab) and added to the cells. For comparison, POLIVY (registered trademark) (manufactured by Genentech) was added at final concentrations of 0, 0.001, 0.01, 0.1, 1, 2, 5, and 10 μg/mL. In addition, unlabeled rituximab was added at a concentration similar to the total antibody concentration added to samples with each radioactivity concentration (0, 0.005, 0.05, 0.15, 0.5, 1.5, 5, 15, 50, 150 μg/mL) and the cells were cultured. After 120 hr from the sample addition, CellTiter-Glo (registered trademark) 2.0 Cell Viability Assay (manufactured by Promega) was added to the medium, chemiluminescence was detected using a microplate reader (SpectraMax i3x, manufactured by Molecular Devices, LLC), and the number of viable cells was calculated. The ratio of the number of viable cells under conditions where no sample was added to the calculated number of viable cells was taken, and the cytotoxic effect was evaluated. The results are shown in Fig. 7. The vertical axis of graph shows the relative value when the number of viable cells under conditions where no antibody was added is set to 1, and the horizontal axis shows the concentration of the added antibody. The graphs show the mean±standard deviation in each group (n=4).

In SU-DHL-2 cells, no cytotoxic effect was confirmed with unlabeled rituximab, and a cytotoxic effect was confirmed with the radioconjugate produced as described in Example 1, at a lower concentration of added antibody than POLIVY (registered trademark).

This application is based on a patent application No. 2021-071320 filed in Japan (filing date: April 20, 2021) and a patent application No. 2022-030389 filed in Japan (filing date: February 28, 2022), the contents of which are incorporated in full herein.

## Claims

1. A conjugate of an anti-CD20 antibody and a chelating agent, wherein the chelating agent is chelated with a metal radionuclide, the anti-CD20 antibody and the chelating agent are linked by a linker (L), and the linker (L) does not contain a thiourea bond.

2. The conjugate according to claim 1, which is a conjugate of an anti-CD20 antibody site-specifically modified with a peptide and a chelating agent, wherein the peptide and the chelating agent are linked by the linker (L).

3. The conjugate according to claim 1, wherein the chelating agent is DOTAGA (α-(2-Carboxyethyl)-1,4,7,10-tetraazacyclododecane-1,4,7,10-tetraacetic acid).

4. The conjugate according to claim 2, wherein the peptide is an amino acid sequence consisting of 13 to 17 amino acid residues and is represented by the following formula:

$$(Xa)\ \text{-}Xaa_1\text{-}\ (Xb)\ \text{-}Xaa_2\text{-}\ (Xc)\ \text{-}Xaa_3\text{-}\ (Xd) \qquad (i)$$

in the formula (i), Xa, Xb, Xc and Xd are continuous X in the number of a, continuous X in the number of b, continuous X in the number of c, and continuous X in the number of d, respectively,
X is an amino acid residue having neither a thiol group nor a haloacetyl group in the side chain,
a, b, c and d are each independently an integer of not less than one and not more than 5, and satisfy $a+b+c+d \leq 14$,
$Xaa_1$ and $Xaa_3$ are each independently an amino acid residue derived from an amino acid having a thiol group in the side chain, or an amino acid residue derived from an amino acid having a haloacetyl group in the side chain, provided that one of $Xaa_1$ and $Xaa_3$ is an amino acid residue derived from an amino acid having a thiol group in the side chain,
$Xaa_1$ and $Xaa_3$ are connected to form a ring structure, and
$Xaa_2$ is a lysine residue, an arginine residue, a cysteine residue, an aspartic acid residue, a glutamic acid residue, 2-aminosuberic acid, or diamino propionic acid, and modified with a crosslinking agent.

5. The conjugate according to claim 1, wherein the metal radionuclide is Zr-89, Y-90, Lu-177, or Ac-225.

6. The conjugate according to claim 1, wherein the linker (L) comprises the formula (10a), the formula (10b), or the formula (10c):

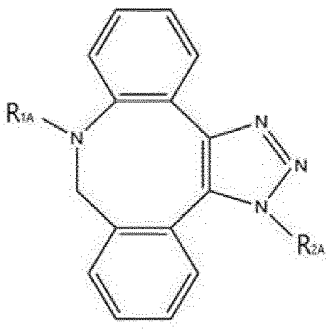

(10a)

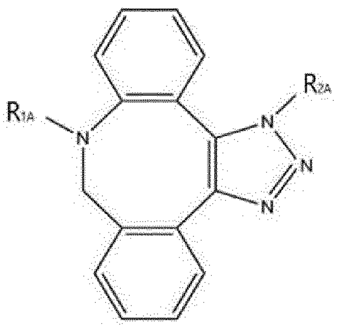

(10b)

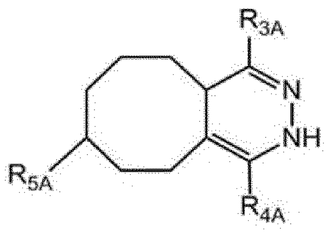

(10c)

In the formula (10a) and the formula (10b), $R_{1A}$ is a binding site with a chelating agent, and $R_{2A}$ is a binding site with the aforementioned antibody or the aforementioned peptide. In the formula (10c), one of $R_{3A}$ and $R_{4A}$ is a hydrogen atom, a methyl group, a phenyl group or a pyridyl group, and the other is a binding site with the aforementioned chelating agent, and $R_{5A}$ is a binding site with the aforementioned antibody or the aforementioned peptide.

7. The radioconjugate according to claim 6, comprising a polyethylene glycol group between the linkage site with the antibody or the peptide and the antibody or the peptide.

8. The radioconjugate according to claim 2, which is conjugated by a click reaction of an anti-CD20 antibody site-specifically modified with the peptide having an azide group introduced into the N-terminal, and a radioactive metal complex of DOTAGA-DBCO represented by the following formula:

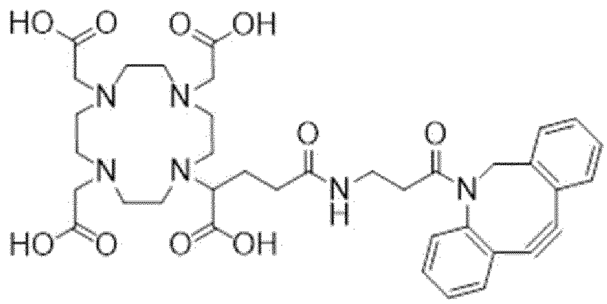

DOTAGA-DBCO

9. The conjugate according to claim 1, wherein the anti-CD20 antibody is rituximab.

10. A radiopharmaceutical comprising the conjugate according to any one of claims 1 to 9 as an active ingredient.

11. The radiopharmaceutical according to claim 10, which is used in a radionuclide therapy for cancer.

12. The radiopharmaceutical according to claim 10, which is used in cancer diagnosis.

13. The radiopharmaceutical according to claim 12, which is used in combination with the radionuclide therapy for cancer using the radiopharmaceutical according to claim 11.

14. A radiopharmaceutical comprising a conjugate of a chelating agent chelated with a metal radionuclide and an anti-CD20 antibody as an active ingredient and satisfying the following condition (1) or (2), wherein the linkage between the anti-CD20 antibody and the chelating agent does not contain a thiourea bond:

(1) the metal radionuclide is $^{177}Lu$ or $^{90}Y$, and the conjugate has a radiochemical purity of not less than 90% when stored at room temperature for 7 days
(2) the metal radionuclide is $^{225}Ac$, and the conjugate has a radiochemical purity of not less than 90% when stored at room temperature for 14 days.

15. A radiopharmaceutical comprising a conjugate of a chelating agent chelated with a metal radionuclide and an anti-CD20 antibody as an active ingredient, wherein the linkage between the anti-CD20 antibody and the chelating agent does not contain a thiourea bond, and the conjugate has a radiochemical purity of not less than 90% at the time of expiry of a period that is a multiple of not less than 1 and not more than 5 of the half-life, based on the half-life of the metal radionuclide.

[Fig. 1]

Light Chain

1 Gln—Ile—Val—Leu—Ser—Gln—Ser—Pro—Ala—Ile—

11 Leu—Ser—Ala—Ser—Pro—Gly—Glu—Lys—Val—Thr—

21 Met—Thr—Cys—Arg—Ala—Ser—Ser—Ser—Val—Ser—

31 Tyr—Ile—His—Trp—Phe—Gln—Gln—Lys—Pro—Gly—

41 Ser—Ser—Pro—Lys—Pro—Trp—Ile—Tyr—Ala—Thr—

51 Ser—Asn—Leu—Ala—Ser—Gly—Val—Pro—Val—Arg—

61 Phe—Ser—Gly—Ser—Gly—Ser—Gly—Thr—Ser—Tyr—

71 Ser—Leu—Thr—Ile—Ser—Arg—Val—Glu—Ala—Glu—

81 Asp—Ala—Ala—Thr—Tyr—Tyr—Cys—Gln—Gln—Trp—

91 Thr—Ser—Asn—Pro—Pro—Thr—Phe—Gly—Gly—Gly—

101 Thr—Lys—Leu—Glu—Ile—Lys—Arg—Thr—Val—Ala—

111 Ala—Pro—Ser—Val—Phe—Ile—Phe—Pro—Pro—Ser—

121 Asp—Glu—Gln—Leu—Lys—Ser—Gly—Thr—Ala—Ser—

131 Val—Val—Cys—Leu—Leu—Asn—Asn—Phe—Tyr—Pro—

141 Arg—Glu—Ala—Lys—Val—Gln—Trp—Lys—Val—Asp—

151 Asn—Ala—Leu—Gln—Ser—Gly—Asn—Ser—Gln—Glu—

161 Ser—Val—Thr—Glu—Gln—Asp—Ser—Lys—Asp—Ser—

171 Thr—Tyr—Ser—Leu—Ser—Ser—Thr—Leu—Thr—Leu—

181 Ser—Lys—Ala—Asp—Tyr—Glu—Lys—His—Lys—Val—

191 Tyr—Ala—Cys—Glu—Val—Thr—His—Gln—Gly—Leu—

201 Ser—Ser—Pro—Val—Thr—Lys—Ser—Phe—Asn—Arg—

211 Gly—Glu—Cys 213

1-128: derived from mouse (underlined)

129-213: derived from human

[Fig. 2-1]

Heavy Chain

1 Gln—Val—Gln—Leu—Gln—Gln—Pro—Gly—Ala—Glu—

11 Leu—Val—Lys—Pro—Gly—Ala—Ser—Val—Lys—Met—

21 Ser—Cys—Lys—Ala—Ser—Gly—Tyr—Thr—Phe—Thr—

31 Ser—Tyr—Asn—Met—His—Trp—Val—Lys—Gln—Thr—

41 Pro—Gly—Arg—Gly—Leu—Glu—Trp—Ile—Gly—Ala—

51 Ile—Tyr—Pro—Gly—Asn—Gly—Asp—Thr—Ser—Tyr—

61 Asn—Gln—Lys—Phe—Lys—Gly—Lys—Ala—Thr—Leu—

71 Thr—Ala—Asp—Lys—Ser—Ser—Ser—Thr—Ala—Tyr—

81 Met—Gln—Leu—Ser—Ser—Leu—Thr—Ser—Glu—Asp—

91 Ser—Ala—Val—Tyr—Tyr—Cys—Ala—Arg—Ser—Thr—

101 Tyr—Tyr—Gly—Gly—Asp—Trp—Tyr—Phe—Asn—Val—

111 Trp—Gly—Ala—Gly—Thr—Thr—Val—Thr—Val—Ser—

121 Ala—Ala—Ser—Thr—Lys—Gly—Pro—Ser—Val—Phe—

131 Pro—Leu—Ala—Pro—Ser—Ser—Lys—Ser—Thr—Ser—

141 Gly—Gly—Thr—Ala—Ala—Leu—Gly—Cys—Leu—Val—

151 Lys—Asp—Tyr—Phe—Pro—Glu—Pro—Val—Thr—Val—

[Fig. 2-2]

161 Ser—Trp—Asn—Ser—Gly—Ala—Leu—Thr—Ser—Gly—

171 Val—His—Thr—Phe—Pro—Ala—Val—Leu—Gln—Ser—

181 Ser—Gly—Leu—Tyr—Ser—Leu—Ser—Ser—Val—Val—

191 Thr—Val—Pro—Ser—Ser—Ser—Leu—Gly—Thr—Gln—

201 Thr—Tyr—Ile—Cys—Asn—Val—Asn—His—Lys—Pro—

211 Ser—Asn—Thr—Lys—Val—Asp—Lys—Lys—Ala—Glu—

221 Pro—Lys—Ser—Cys—Asp—Lys—Thr—His—Thr—Cys—

231 Pro—Pro—Cys—Pro—Ala—Pro—Glu—Leu—Leu—Gly—

241 Gly—Pro—Ser—Val—Phe—Leu—Phe—Pro—Pro—Lys—

251 Pro—Lys—Asp—Thr—Leu—Met—Ile—Ser—Arg—Thr—

261 Pro—Glu—Val—Thr—Cys—Val—Val—Val—Asp—Val—

271 Ser—His—Glu—Asp—Pro—Glu—Val—Lys—Phe—Asn—

281 Trp—Tyr—Val—Asp—Gly—Val—Glu—Val—His—Asn—

291 Ala—Lys—Thr—Lys—Pro—Arg—Glu—Glu—Gln—Tyr—

301 Asn*—Ser—Thr—Tyr—Arg—Val—Val—Ser—Val—Leu—

311 Thr—Val—Leu—His—Gln—Asp—Trp—Leu—Asn—Gly—

321 Lys—Glu—Tyr—Lys—Cys—Lys—Val—Ser—Asn—Lys—

331 Ala—Leu—Pro—Ala—Pro—Ile—Glu—Lys—Thr—Ile—

[Fig. 2-3]

341 Ser—Lys—Ala—Lys—Gly—Gln—Pro—Arg—Glu—Pro—

351 Gln—Val—Tyr—Thr—Leu—Pro—Pro—Ser—Arg—Asp—

361 Glu—Leu—Thr—Lys—Asn—Gln—Val—Ser—Leu—Thr—

371 Cys—Leu—Val—Lys—Gly—Phe—Tyr—Pro—Ser—Asp—

381 Ile—Ala—Val—Glu—Trp—Glu—Ser—Asn—Gly—Gln—

391 Pro—Glu—Asn—Asn—Tyr—Lys—Thr—Thr—Pro—Pro—

401 Val—Leu—Asp—Ser—Asp—Gly—Ser—Phe—Phe—Leu—

411 Tyr—Ser—Lys—Leu—Thr—Val—Asp—Lys—Ser—Arg—

421 Trp—Gln—Gln—Gly—Asn—Val—Phe—Ser—Cys—Ser—

431 Val—Met—His—Glu—Ala—Leu—His—Asn—His—Tyr—

441 Thr—Gln—Lys—Ser—Leu—Ser—Leu—Ser—Pro—Gly—

451 Lys

1-150: derived from mouse (underlined), 151-451: derived from human *: sugar chain binding site

basic sugar chain structure:

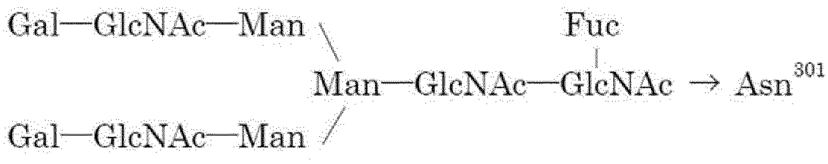

[Fig. 3]

antigen binding activity ($^{225}$Ac-ARG)

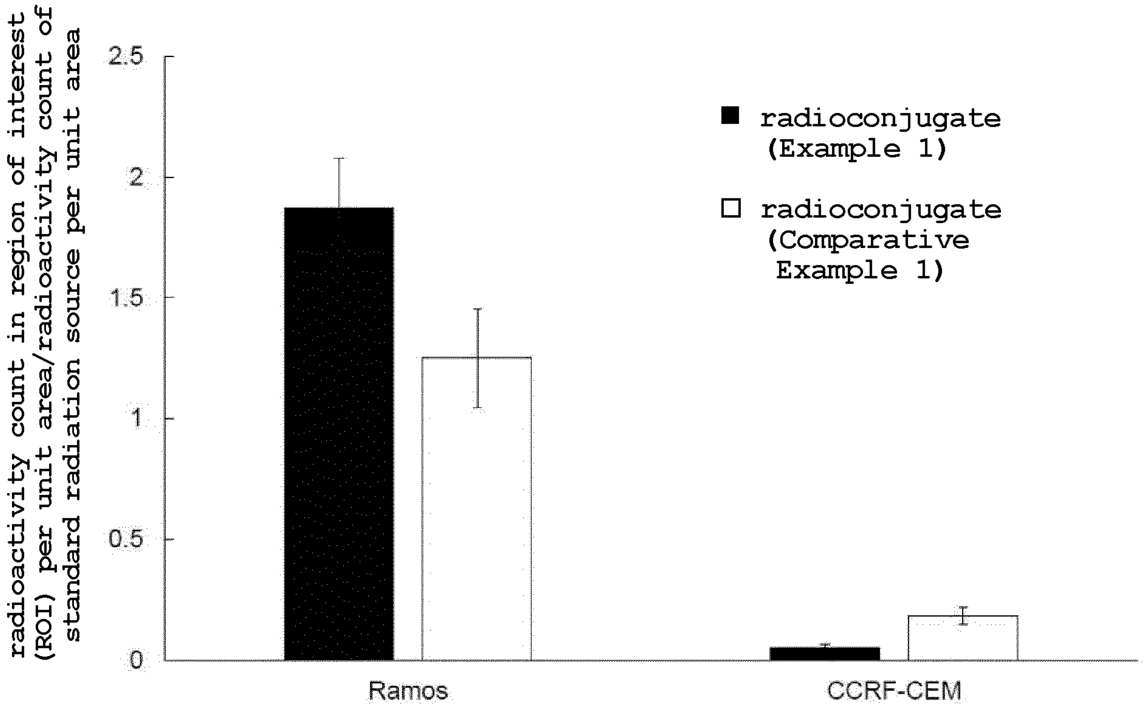


[Fig. 4]

efficacy evaluation ($^{225}$Ac therapy experiment)

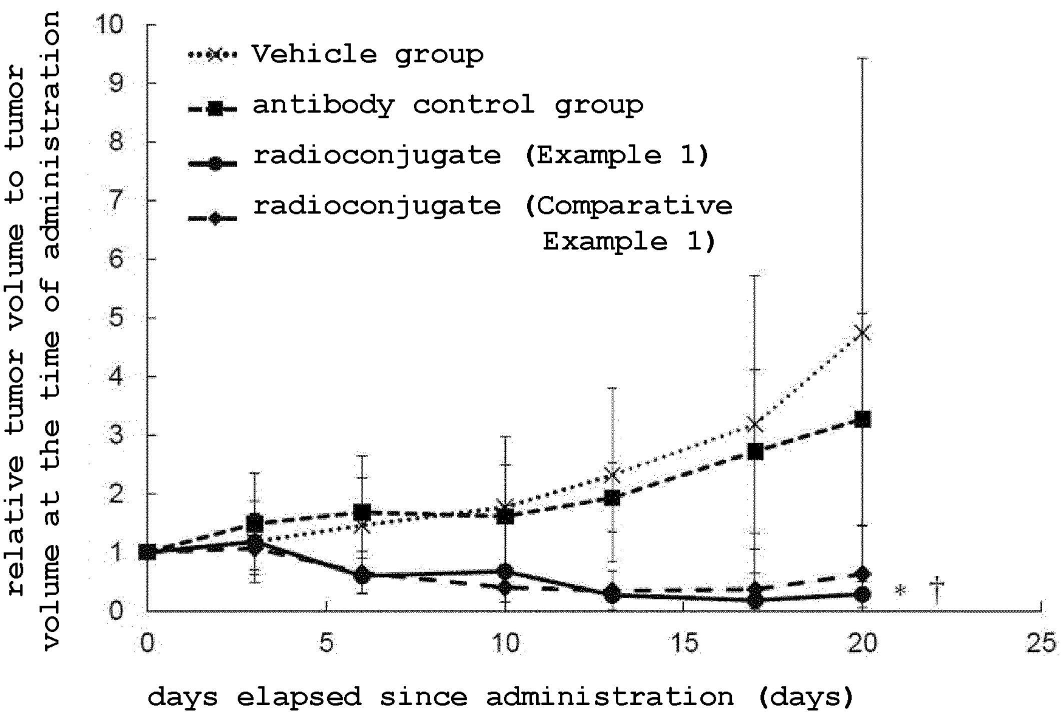

[Fig. 5]

antigen binding activity ($^{89}$Zr-ARG)

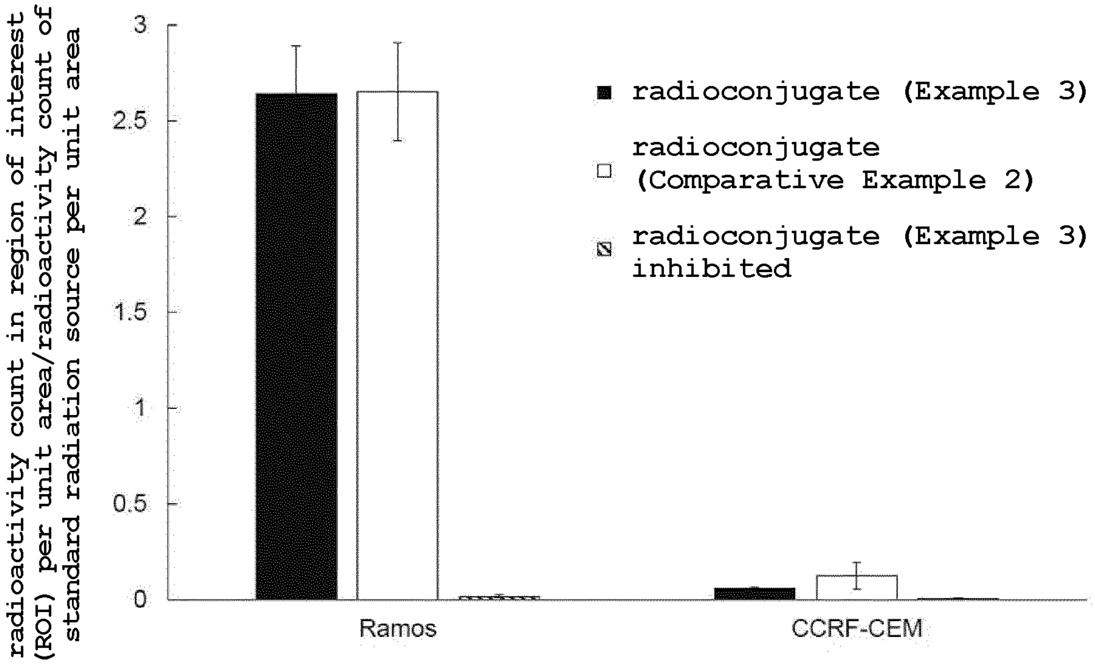


[Fig. 6]

efficacy evaluation ($^{89}$Zr tumor accumulation)

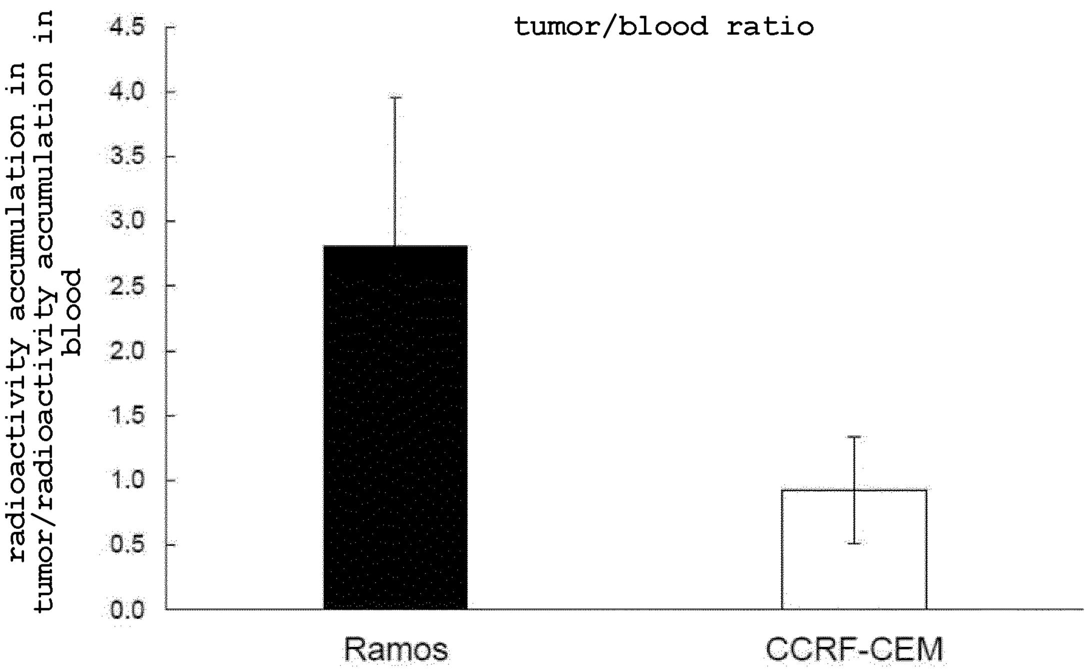

[Fig. 7]

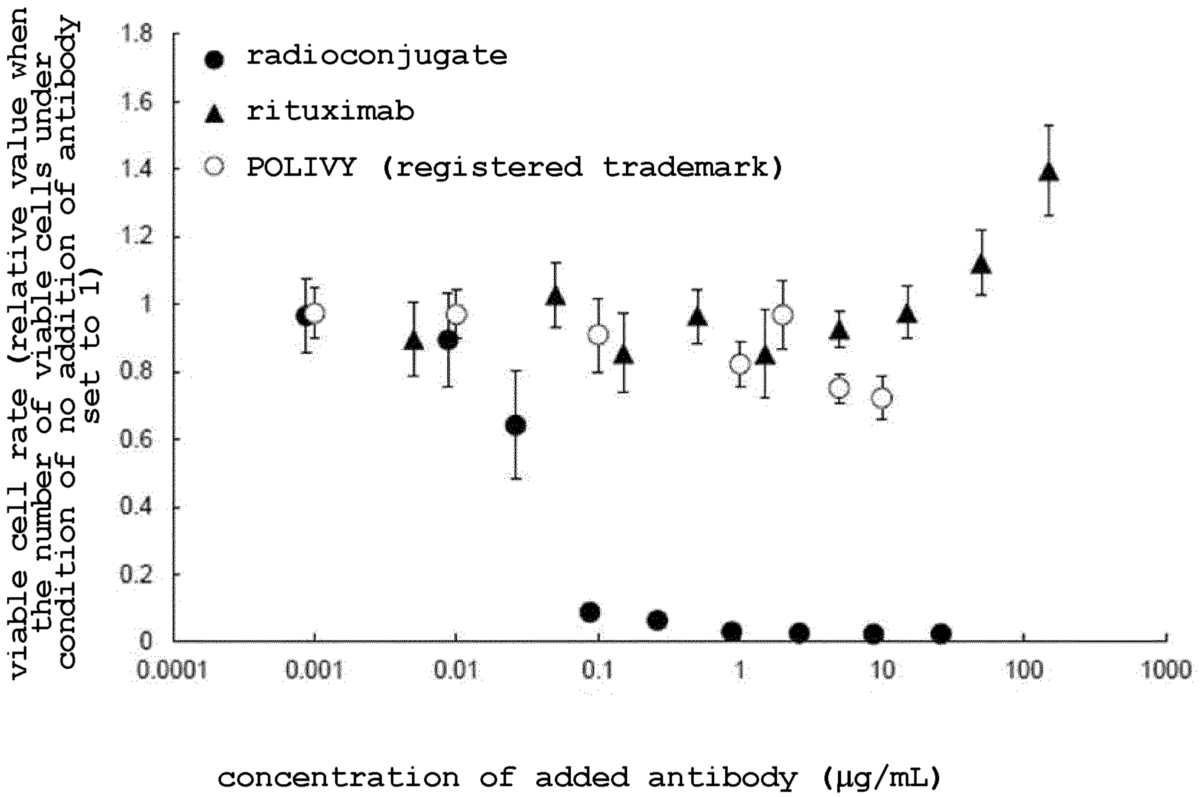
viable cell rate (relative value when the number of viable cells under condition of no addition of antibody set to 1)
1.8
1.6
1.4
1.2
1
0.8
0.6
0.4
0.2
0
radioconjugate
rituximab
POLIVY (registered trademark)
0.0001
0.001
0.01
0.1
1
10
100
1000
concentration of added antibody (μg/mL)

## INTERNATIONAL SEARCH REPORT

International application No. **PCT/JP2022/018246**

**A. CLASSIFICATION OF SUBJECT MATTER**

***A61K 47/68***(2017.01)i; ***A61K 39/395***(2006.01)i; ***A61K 45/00***(2006.01)i; ***A61K 47/60***(2017.01)i; ***A61K 47/69***(2017.01)i; ***A61K 51/00***(2006.01)i; ***A61K 51/02***(2006.01)i; ***A61K 51/10***(2006.01)i; ***A61P 35/00***(2006.01)i; ***C07K 4/00***(2006.01)i; ***C07K 16/28***(2006.01)i

FI: A61K47/68 ZNA; A61K39/395 D; A61K39/395 N; A61K39/395 Y; A61K45/00; A61K47/60; A61K47/69; A61K51/00 100; A61K51/00 200; A61K51/02 100; A61K51/10 100; A61K51/10 200; A61P35/00; C07K4/00; C07K16/28 ZNA

According to International Patent Classification (IPC) or to both national classification and IPC

**B. FIELDS SEARCHED**

Minimum documentation searched (classification system followed by classification symbols)

A61K47/68; A61K39/395; A61K45/00; A61K47/60; A61K47/69; A61K51/00; A61K51/02; A61K51/10; A61P35/00; C07K4/00; C07K16/28

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
|---|---|---|
| Y | FORRER, F. et al. Radioimmunotherapy with 177Lu-DOTA-Rituximab: Final Results of a Phase I/II Study in 31 Patients with Relapsing Follicular, Mantle Cell, and Other Indolent B-Cell Lymphomas. THE JOURNAL OF NUCLEAR MEDICINE. 2013, vol. 54, no. 7, pp. 1045-1052<br>title, abstract, p. 1046, left column, lines 8-4 from the bottom, fig. 1, 2, table 4 | 1-15 |
| Y | DAHLE, J. et al. Initial evaluation of 227Th-p-benzyl-DOTA-rituximab for low-dose rate alpha-particle radioimmunotherapy. Nuclear Medicine and Biology. 2006, vol. 33, pp. 271-279<br>title, abstract, p. 272, right column, last paragraph | 1-15 |
| Y | XIE, Q. et al. Establishing Reliable Cu-64 Production Process: From Target Plating to Molecular Specific Tumor Micro-PET Imaging. Molecules. 2017, vol. 22, 641, pp. 1-10<br>title, abstract, p. 8, 2nd paragraph | 1-15 |
| Y | WO 2019/203191 A1 (NIHON MEDIPHYSICS CO LTD) 24 October 2019 (2019-10-24)<br>claims 1-3, 6, 7, paragraphs [0001]-[0020] | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **11 July 2022** | **19 July 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2022/018246**

**C. DOCUMENTS CONSIDERED TO BE RELEVANT**

| Category* | Citation of document, with indication, where appropriate, of the relevant passages | Relevant to claim No. |
|---|---|---|
| Y | MITRAN, B. et al. Trastuzumab cotreatment improves survival of mice with PC-3 prostate cancer xenografts treated with the GRPR antagonist 177Lu-DOTAGA-PEG2-RM26. Int. J. Cancer. 2019, vol. 145, pp. 3347-3358<br>title, p. 3350, left column, line 11 from the bottom to right column, line 21, table 1, fig. 4 | 1-15 |
| P, Y | WO 2021/075546 A1 (NIHON MEDIPHYSICS CO LTD) 22 April 2021 (2021-04-22)<br>entire text | 1-15 |
| P, Y | WO 2021/075544 A1 (NIHON MEDIPHYSICS CO LTD) 22 April 2021 (2021-04-22)<br>entire text | 1-15 |

Form PCT/ISA/210 (second sheet) (January 2015)

**INTERNATIONAL SEARCH REPORT**

International application No.
**PCT/JP2022/018246**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2022/018246**

| Patent document cited in search report | Publication date (day/month/year) | Patent family member(s) | Publication date (day/month/year) |
|---|---|---|---|
| WO 2019/203191 A1 | 24 October 2019 | US 2021/0170058 A1<br>claims 1-3, 6, 7, paragraphs [0001]-[0044]<br>EP 3783016 A1<br>CN 112041337 A<br>KR 10-2020-0143366 A | |
| WO 2021/075546 A1 | 22 April 2021 | (Family: none) | |
| WO 2021/075544 A1 | 22 April 2021 | US 2021/0338852 A1<br>entire text | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2004032828 A
- JP 2006511532 A
- US 5736137 A
- WO 2016186206 A
- WO 2017217347 A
- WO 2018230257 A
- WO 2021080008 A
- JP 2021071320 A
- JP 2022030389 A

### Non-patent literature cited in the description

- **BERNHARD et al.** DOTAGA-Anhydride: A Valuable Building Block for the Preparation of DOTA-Like Chelating Agents. *Chem. Eur. J.,* 2012, vol. 18, 7834-7841